# EUROPEAN PATENT APPLICATION

(11) **EP 4 835 480 A2**
(43) Date of publication of application: **04.11.2026**
(21) Application number: 26191879.1
(22) Date of filing: 04.04.2023
(51) Int. Cl.: G01N 33/92

(54) **MASS SPECTROMETRY METHODS FOR CANCER DIAGNOSIS**

(30) Priority: 04.04.2022 US 202263326974 P
(62) Document number(s) of the earlier application(s) in accordance with Art. 76 EPC: 23785559.8 / 4 504 250
(71) Applicant: AOA Dx, Boulder, CO 80302 (US)
(72) Inventor: SARAGOVI, Horacio U., Montreal, Québec H4A3L8 (CA)
(74) Representative: Hoefer & Partner Patentanwälte mbB

(57) **Abstract**

The present disclosure relates to mass spectrometry methods for cancer diagnosis and prognosis.

## Description

### Background of the Invention

Cancer involves abnormal cell growth with the potential to invade or spread to other parts of the body. Despite decades of cancer research, cancer continues to cause a significant number of deaths (~1,600 deaths per day in the U.S. in 2020) largely due to a lack of means for early and/or accurate detection. For example, ovarian cancer is the most lethal gynecologic cancer and the third cause of death among women**.** Serous Ovarian Cancer is the most aggresive subtype among ovarian cancer and it has been commonly labelled as the "silent killer" because most patients are diagnosed at advanced stages. Symptoms are vague and easily confounded with other conditions. Currently, CA-125 is a blood marker used both to diagnose and to monitor treatment efficacy. Although CA-125 levels are raised in 80% of epithelial tumors, most of these tumors are in an advanced stage. Serum CA-125 has a low detection rate for early diagnosis because its levels are elevated in <50% of stage I ovarian cancers, and has limited specificity. Thus, there is a great need for new biomarkers, e.g., in cancer tissue and in blood, to detect early stages of cancer, which would improve the survival of cancer patients.

### Summary of the Invention

Gangliosides are glycolipids that comprise (i) a carbohydrate structure that specifically defines it by name, and (ii) lipid tails that can vary in carbon chain length. The present invention is based, at least in part, on the discovery that gangliosides (e.g., GD2, GD3, GM2, GD1b, or lipoforms thereof) are useful biomarkers for cancer diagnosis and prognosis, especially for early detection of cancer.

Provided herein are methods for cancer diagnosis and prognosis. For example, provided herein are mass spectrometry-based methods for detecting and measuring the amount of a ganglioside, a lipoform of a ganglioside, as well as the lipid length of a ganglioside. In addition, provided herein are methods of diagnosing a cancer using a liquid biopsy (e.g., blood, serum) or solid tissue biopsy (e.g., cancer tissue). The surprising and unexpected prevalence of certain gangliosides, lipoforms of gangliosides, and/or lipid length of gangliosides, provides a generation of novel biomarkers for cancer diagnosis and prognosis.

### Brief Description of Figures

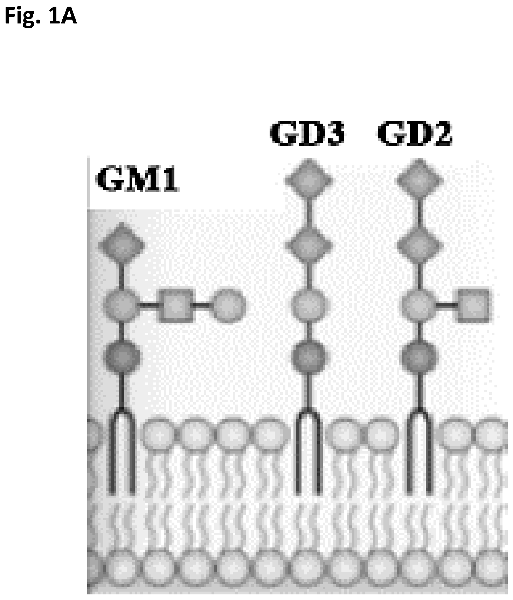

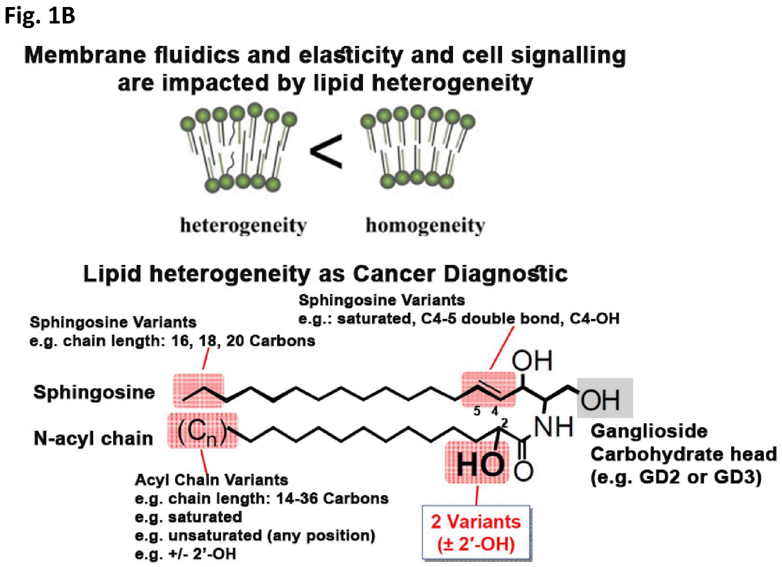

**Fig. 1A****-****Fig. 1B** show a schematic diagram of gangliosides. Fig. 1A shows a schematic diagram of GD2 and GD3 gangliosides. Each geometric shape is a type of sugar. The exposed glycan tree is linked to a ceramide, which is linked to two lipid tails. The sugar head of normal GM1 and the tumor GD3 differ by 2 sugars, and GD2 and GD3 differ from each other by 1 sugar. GD2 is a biosynthetic product of GD3, so the GD2 and GD3 can be often (but not always) detected on the same cell. Fig. 1B shows a representation of how certain lipoforms can assemble differently on the membrane of cells, depending on lipid homogeneity or heterogeneity, resulting in different biological signals or biological events. Fig 1B also shows a representation of how the Sphingosine chain can vary in Carbon length, and at the saturation at the Carbons positions 4-5. The acyl chain can vary more significantly in Carbon length, the (mono)saturation of 2 Carbons, and the hydroxylation of the 2-Carbon position.

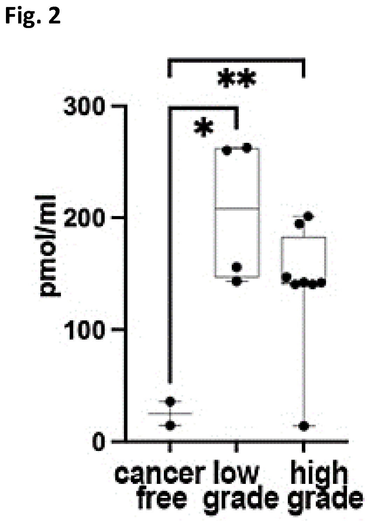

**Fig. 2** shows LC-MS/MS detection of GD3 in ovarian cancer. All GD3 species are shown combined, regardless of lipoforms, ANOVA, post-hoc Holm Sidak, * p<0.05, ** p<0.01. GD3 analytes in early and late stage ovarian cancer (n=13; 4 early stage and 9 late stage) versus non-cancer (n=2). The ovarian cancer data are absolute quantification (pmol/ml). Glycolipids measured in serum that was taken at the time of diagnosis. Cancer-free samples have very low total GD3, and cancer samples have significantly higher total GD3.

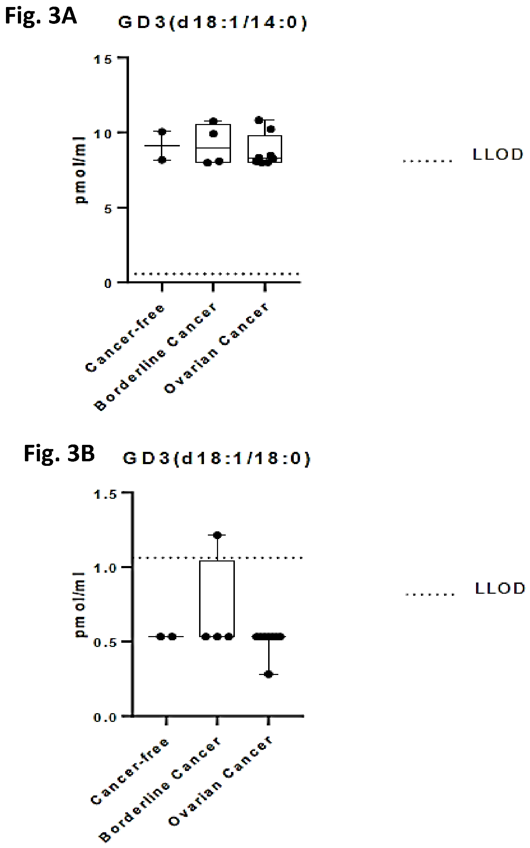

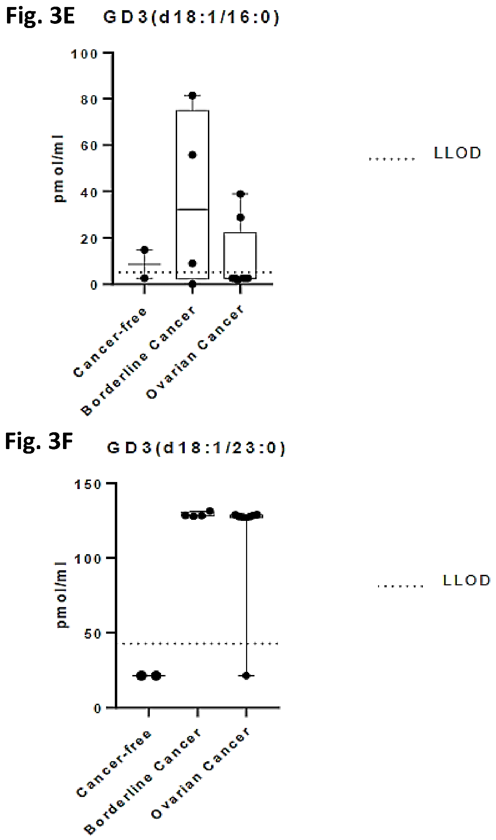

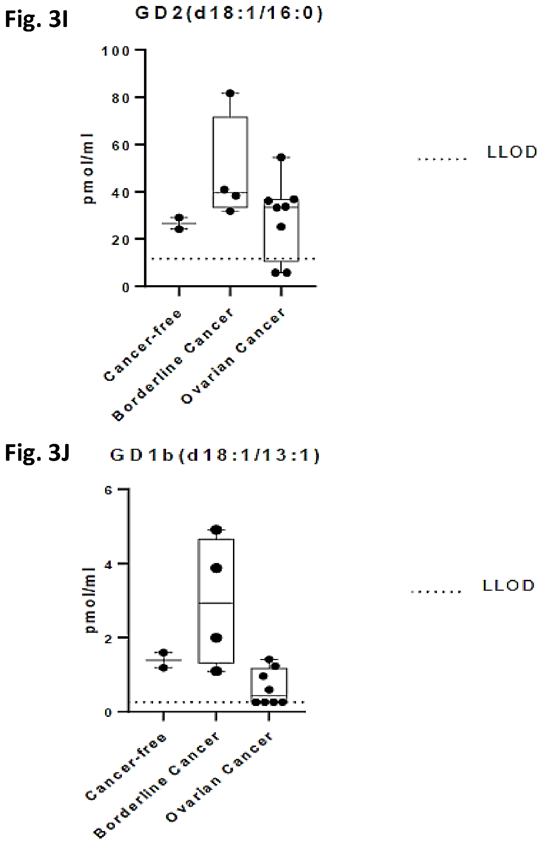

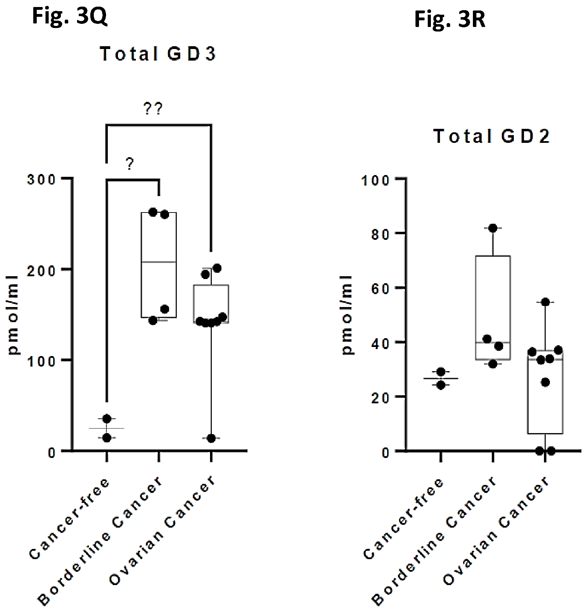

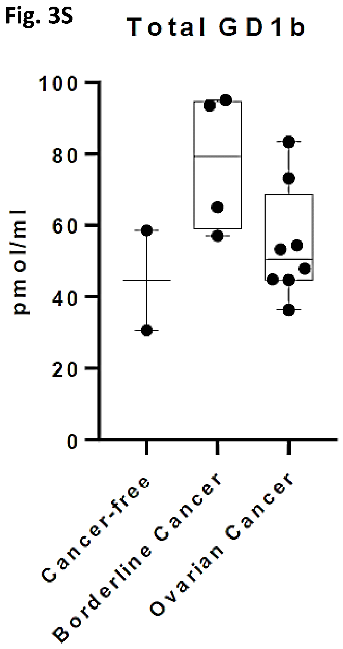

**Fig. 3A-3S** show LC-MS/MS detection of lipoforms of gangliosides in ovarian cancer. Fig. 3A-Fig. 3H show lipoforms of GD3. Fig. 3I shows a lipoform of GD2. Fig. 3J-Fig. 3P show lipoforms of GD1b. Fig. 3Q shows total GD3 detected. Fig. 3R and Fig. 3S show total GD2 and GD1b detected, respectively. Certain lipoforms show a significant increase in cancer samples vs. non-cancer samples. For example, Fig. 3F demonstrates that GD3(d18:1/23:0) is significantly increased in ovarian cancer samples, thereby providing an excellent marker for cancer diagnosis/prognosis.

### Detailed Description of the Invention

Gangliosides are a family of >40 different sialic acid-containing glycosphingolipids. Each glycan tree is structurally unique and defines each ganglioside by name. Some gangliosides such as GM1 are normal and ubiquitous. Other gangliosides such as GD2 and GD3 are tumor-markers (Tumor-Marker Gangliosides; also referred to as TMGs). They are low/absent in normal cells, and are expressed at high levels in cancer. Hence, GD2 and GD3 are etiological biomarkers (i.e., those with indispensable function for the cancer), which are preferred because cancers do not easily downregulate expression of the marker.

GD2 and GD3 regulate membrane fluidity, raft size, and function, and provide tumors with advantages in growth/metastasis, immune evasion, and blockade. The lipid tails are embedded in the outer leaflet of cell membranes and are variable in length.

GD2 or GD3 provide for stable, invariant, and non-mutating targets, expression is conserved across mammalian species (the glycan tree is identical), expression is homogeneous and uniform in cell lines and in primary tumors, and density of expression does not downregulate in the surviving tumor cells after chemotherapy. In addition to being present on the tumor cell surface, GD2 and GD3 can be shed into the extracellular environment.

However, studies of expression of GD2 and GD3 in tissues or in circulation have only been reported in a few patient samples. Assays to detect GD2 or GD3 that may be expressed in tissue or in serum are not quantitative or standardized; only yield estimations; and resulted in contradictory conclusions. Since GD2 and GD3 are glycolipids and the products can be generated via multiple biosynthetic pathways and enzymes, monitoring mutations or mRNA expression is not feasible. GD2 and GD3 therefore remain underexploited for diagnosis of cancer.

The present disclosure is based, at least in part, on the discovery that mass spectrometry-based methods provide powerful means to determine accurately the gangliosides associated with cancer, e.g., tumors. Further provided herein are diagnostic methods that employ novel biomarkers of gangliosides and their lipoforms. These novel biomarkers show surprising and unexpected prevalence in certain cancer types, e.g., ovarian cancer. Detection of such biomarker(s) provides sensitive and accurate diagnostic methods for cancer, especially at an early stage of cancer.

### Definitions

The articles "a" and "an" are used herein to refer to one or to more than one (*i.e.* to at least one) of the grammatical object of the article. By way of example, "an element" means one element or more than one element.

The term "borderline tumor" is art-recognized, and refers to an atypical proliferative tumor with relatively lower malignant potential. For example, borderline ovarian tumors are atypical proliferative tumors with relatively lower malignant potential as compared to e.g., ovarian cancer (see below).

The lipid length refers to the length of the lipid tails of a ganglioside that are important for diagnostics. The lipid tails are embedded in the outer leaflet of cell membranes. The lipids can be variable in length. Notably, these differences occur when comparing amongst gangliosides as well as within a single ganglioside. Thus, the lipid heterogeneity and changes thereof are important for diagnostics and methods described herein.

The term "lipoform" of a ganglioside refers to a variant of a ganglioside that differs in its lipid (see e.g., Kolter (2012) *ISRN Biochem* 506160).

The term "minimal residual disease" is art recognized, and is used to describe a small number of cancer cells in the body during or after cancer treatment, when the patient is in remission. The number of remaining cells may be so small that they do not cause any physical signs or symptoms and often cannot even be detected through traditional methods. It is a major cause of relapse of cancer.

The term "neoadjuvant therapy" refers to a treatment given before the primary treatment. Examples of neoadjuvant therapy can include chemotherapy, radiation therapy, and hormone therapy.

The term "preventing" is art-recognized, and when used in relation to a condition, such as a viral/bacterial infection or a disease such as cancer is well understood in the art, and includes administration of a treatment, e.g., a composition which reduces the frequency of, or delays the onset of, symptoms of a medical condition in a subject relative to a subject which does not receive the treatment. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount.

The term "remission" is art recognized, and refers to a condition in which the signs and symptoms of the cancer are reduced.

As used herein, "subject" refers to any healthy animal, mammal or human, or any animal, mammal or human afflicted with a cancer. The term "subject" is interchangeable with "patient". The term "non-human animal" includes all vertebrates, *e.g.*, mammals and non-mammals, such as non-human primates, sheep, dog, cow, chickens, amphibians, reptiles, etc.

A "therapeutically effective amount" of a compound is an amount capable of producing a medically desirable result in a treated patient, e.g., induce immune response against a ganglioside, decrease tumor burden, decrease the growth of tumor cells, or alleviate any symptom associated with cancer, with an acceptable benefit: risk ratio, preferably in a human or non-human mammal.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (*e.g.*, disease or other unwanted state of the host animal), then the treatment is prophylactic (*i.e.*, it protects the host against developing the unwanted condition); whereas, if it is administered after manifestation of the unwanted condition, the treatment is therapeutic (*i.e.*, it is intended to diminish, ameliorate, or stabilize the existing unwanted condition or side effects thereof).

### Gangliosides

Gangliosides are glycosphingolipids comprising one or more sialic acids. Glycosphingolipids contain a hydrophobic ceramide or sphingoid lipid tail, which is usually anchored to the outer leaflet of the plasma membrane. They also contain an oligosaccharide moiety and are classified according to this carbohydrate structure (ganglio, isoganglio, lacto etc.). Gangliosides are an important subclass of glycosphingolipids, because they contain negatively charged sialic acids (N-acetylneuraminic acid or N-glycolylneuraminic acid) linked to the lipooligosaccharide moiety. Gangliosides are named and classified according to the number of sialic acid residues attached (M for one, D for two, T for 3 and Q for 4) to the inner sugar moiety and according to their chromatographic mobility. The numbering of the gangliosides (5-x) is based on the number (x) of the inner sugar moieties (glucose, galactose or GalNAc) according to the original experimental classification of Svennerholm. Thus, if x is 4, the gangliosides are: GM1, GD1, GT1, x = 3 for GM2, GD2, GT2, and x = 2 for GM3, GD3, and GT3.

Heterogeneity is not only found within the glycan part, but also within the ceramide moiety. This can consist of different sphingoid bases, sphinganine, sphingosine, and phytosphingosine of different chain lengths, which can be further modified by O-acetylation. In higher animals, C18- and C20-sphingosine are the most abundant sphingoid bases of gangliosides.

The fatty acids found in the ceramide part of gangliosides are mostly saturated. α-Hydroxylated fatty acids are not frequently found in brain gangliosides, but are, for example, abundant in gangliosides from intestine, liver, or kidney, and in GM4. To specify the lipoform of a ganglioside, designations such as (d18:1/18:0)GM3 are used for a II³Neu5AcLacCer with a sphingosine (d = dihydroxy, 1 = one double bond) of 18 carbons and a stearoyl residue (18:0) within the ceramide portion (see Kolter (2012) ISRN Biochem 506160). The functional consequences of the heterogeneities in the lipid component are largely unknown, but the lipid part can mask the receptor function of ganglioside glycans via interaction with membrane cholesterol.

As presented herein, gangliosides are tumor biomarkers. In some embodiments, a tumor-associated ganglioside, also referred to as a "tumor marker ganglioside" or a TMG, is selected from GD2, GD3, GD1b, GT1b, fucosyl-GM1, GloboH, polysialic acid (PSA), GM2, GM3, sialyl-Lewis^{X}, sialyl-Lewis^{Y}, sialyl-Lewis^{A}, sialyl-Lewis^{B}, Lewis^{Y}, any portion thereof, and modified version thereof. In preferred embodiments, the ganglioside is GD2, GD3, GM2, GD1b, or lipoforms thereof.

Accordingly, as used herein, the term ganglioside may refer to a ganglioside, a tumor-associated ganglioside, a portion thereof, a lipoform thereof, a glycan variant thereof, or any other isoform/variant thereof.

### Analyzing / Detecting Gangliosides

A ganglioside biomarker can be analyzed according to the methods described herein and other suitable techniques known in the art. The presence, level, or the lipid length of a ganglioside (e.g., GD1b, GD2, GD3, GM2, or lipoforms thereof) can be detected using methods including, without limitation, immunodiffusion, immunoelectrophoresis, an immunofluorescence assay, an enzyme immunoassay, an immunoprecipitation assay, a chemiluminescence assay, an immunohistochemical assay, a dot blot assay, or a slot blot assay. General techniques to be used in performing the various immunoassays noted above and other variations of the techniques, such as *in situ* proximity ligation assay (PLA), fluorescence polarization immunoassay (FPIA), fluorescence immunoassay (FIA), enzyme immunoassay (EIA), nephelometric inhibition immunoassay (NIA), enzyme linked immunosorbent assay (ELISA), radioimmunoassay (RIA), sandwich ELISA, competitive ELISA, agglutination, complement assays, high performance liquid chromatography (HPLC), thin layer chromatography (TLC), hyperdiffusion chromatography, and the like (*e.g.*, Basic and Clinical Immunology, Sites and Terr, eds., Appleton and Lange, Norwalk, Conn. pp 217-262, 1991 which is incorporated by reference) alone or in combination or alternatively with NMR, MALDI-TOF, LC-MS/MS, gas chromatography and similar methods that are known to those of ordinary skill in the art.

Such techniques can also be used to monitor the ganglioside levels on a cell, in tissues, or in blood/plasma.

In preferred embodiments, a mass spectrometry (e.g., MALDI-TOF, LC-MS/MS, LC-MS, LC-ESI-MS/MS, nano-LC-ESI-MS/MS (also called nLC-ESI-MS/MS or nanobore LC-ESI-MS/MS), or others known in the art) is used to detect the presence, level, or the lipid length of a ganglioside (e.g., GD1b, GD2, GD3, GM2, or lipoforms thereof) in a biological specimen, such as liquid biopsy (e.g., blood, saliva, serum, cell/tissue (e.g., cancer cell); see the section on Samples). The mass spectrometry-based method is particularly useful in determining the heterogeneity of the lipid length of a ganglioside, or certain lipoforms of gangliosides, which are novel biomarkers of the present disclosure.

### Mass Spectrometry

Liquid chromatography coupled with tandem mass spectrometry (LC-MS/MS) has been frequently utilized for the sensitive and selective determination of the trace level compounds in biological samples. In particular, LC-MS/MS equipped with electrospray ionization (ESI) as the ion source is most often used method, since ESI can ionize wide range of the compounds including the polar compounds or large molecular weight compounds. Through the coupling with HPLC, the detected characteristics can be quantified by ultraviolet light or MS signal intensity.

Accordingly, any mass spectrometry-based methods can be applied to the methods of the present disclosure. Exemplary mass spectrometry-based methods include but not limited to LC-MS-based methods (e.g., LC-MS/MS, LCⁿ-MSⁿ), LC-MS-based methods comprising the use of ESI (e.g., LC-ESI-MS, LC-ESI-MS/MS, LC-ESI-CID-MS/MS), and nanobore LC-MS-based methods (e.g., nanobore LC-ESI-MS, nanobore LC-ESI-MS/MS).

In LC-ESI-MS/MS, the analytes having the appropriate hydrophobic structures can be sensitively detected, since (i) the hydrophobic ions prefer to reside at the droplet surface generated by electrospray and these ions enter the gas phase more readily than those in the droplet interior and show the higher signal intensities. (ii) The hydrophobic compounds can be well separated on the reversed phase column from salts and interfering compounds possessing suppression effects on ESI. (iii) The hydrophobic compounds are eluted by the mobile phase with the higher organic solvent content. The higher organic solvent content is suitable for the stable generation of charged droplets by electrospray and thus gives the higher signal intensities (see e.g., Santa (2013) Drug Discoveries & Therapeutics, 7:9-17, which is incorporated by reference in its entirety).

Gangliosides can detected using LC-ESI-MS/MS (see e.g., Fuller et al. (2014) Anal Biochem 458:20-26; Sorensen (2006) Rapid Commun Mass Spectrom 20:3625-33; each of which is incorporated by reference in their entirety). Gangliosides and lipforms can be detected using LC-ESI-MS/MS (see, e.g., Ikeda et al.(2008) J Lipid Res 49:2678-89), which is incorporated by reference in its entirety). LC-ESI-CID-MS/MS has been widely applied to detect gangliosides or glycan sequencing, which shows improved speed and sensitivity.

As indicated above, hydrophobic compounds, e.g., gangliosides or their lipoforms, can be efficiently separated and quantified using a reverse phase column connected to HPLC or UPLC. A reverse phase column, or reversed-phase HPLC columns, are chromatography columns that contain a non-polar stationary phase. A sample is placed into a reverse phase column and then solvent is added to flush the sample through the stationary phase. Because the stationary phase in a reversed-phase HPLC column is non-polar, the polar components of the sample will drain from the column first, followed by the non-polar components. Reversed-phase HPLC columns can be packed or capillary, made of glass or metal, and can have many different hydrophobic substances as the stationary phase.

A number of reverse phase columns are available commercially. For example, various categories of reverse phase columns, such as C18 Reversed Phase LC Columns, Biphenyl Reversed Phase LC Columns, C4 Reversed Phase LC Columns, C8 Reversed Phase Columns, Phenyl Reversed Phase LC Columns, C1 Reversed Phase LC Columns, PFP Reversed Phase LC Columns, C30 Reversed Phase LC Columns, Polar Embedded Reversed Phase LC Columns, Polar Endcapped Reversed Phase LC Columns, Porous Graphitic Carbon Reversed Phase LC Columns, Phenyl-Hexyl Reversed Phase LC Columns, and Alkyl Reversed Phase LC Columns are known in the art and commercially available (Thermo Fisher Scientific, Waltham, MA; Waters Corporation, Milford, MA).

Analysis of biological samples by MS is challenging due to the limited amount of sample available for analysis, the very low concentration of analyte, and the potential for interference from sample matrix. The advent of nanobore or nano LCⁿ /MSⁿ offers a solution to these limitations. The nL/min fl ow rate creates much smaller droplets that are more readily desolvated and result in higher MS sensitivity. In addition, lower detection limits are achieved, less sample is required, and there can be an increased tolerance to chemical interferences compared to conventional LC flow rates. Interfacing nanobore LC to MS utilizes emitters/sprayers that have tips with inner diameters of ~1-30 µm.

Nanobore Liquid Chromatography (LC) coupled with LC-MS is well known in the art (see, e.g., Valaskovic and Kelleher (2002) Curr Top Med Chem 2:1-12; Siu et al. (2009) J Proteome Res 8:3797-807; Harbourt et al. (2012) Ana Chem 84:98-105, which are incorporated by reference in their entirety), and nanobore columns are readily available commercially (see, e.g., BioBasic^{™} 18 Nanobore HPLC column (Thermo Scientific^{™} 72105107563), BioBasic^{™} 8 5 µm Nanobore HPLC Columns, HPLC Column ACE Nanobore, and Thermo Scientific^{®} Hypersil GOLD Nanobore HPLC Columns).

The "level" or "amount" of a biomarker (e.g., a ganglioside or one or more of its lipoforms) in a subject is "significantly" higher or lower than the level of a biomarker in a control (e.g., normal sample), if the amount of the biomarker is greater or less, respectively, than the level in a control by an amount greater than the standard error of the assay employed to assess amount.

In some embodiments, the amount or level of a biomarker in a subject can be considered "significantly" higher or lower than the normal and/or control amount if the amount is at least or about 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, 1000%, 1500%, 2000%, 2500%, 3000%, or more, or any range in between, such as 5%-100%, higher or lower, respectively, than the normal and/or control amount of the biomarker. Such significant modulation values can be applied to any metric described herein, such as the level of a ganglioside or a change in the heterogeneity of the lipid length of a ganglioside, and the like.

The term "heterogeneity of lipid length" of a at least one ganglioside includes the level or distribution pattern of the lipid length of at least one ganglioside in a given sample.

In some embodiments, there is a change (e.g., increase or decrease) or a significant change in the heterogeneity of lipid length, if the level or distribution pattern of the lipid length of at least one ganglioside in a subjection sample is at least or about 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, 1000%, 1500%, 2000%, 2500%, 3000%, or more, or any range in between, such as 5%-100%, higher or lower than that in a normal and/or a control sample.

Similarly, the term "heterogeneity of a lipid length" of a ganglioside includes the distribution patterns of gangliosides having a short lipid length vs. a long lipid length (see Example 6). Gangliosides have two lipid tails: sphingosine and acyl. As used herein, the two lipid tails are not distinguished. Accordingly, the term "heterogeneity of a lipid length" as used herein refers to the average length of both lipid tails of a ganglioside. In some embodiments, there is a change or a significant change in the heterogeneity of a lipid length of a ganglioside, if the amount or level of a ganglioside having a short lipid length (14-24 carbons) in a subject sample is at least or about 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, 1000%, 1500%, 2000%, 2500%, 3000%, or more, or any range in between, such as 5%-100%, higher or lower than that in a normal and/or a control sample.

In some embodiments, there is a change or a significant change in the heterogeneity of a lipid length of a ganglioside, if the amount or level of a ganglioside having a long lipid length (26-38 carbons) in a subject sample is at least or about 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, 1000%, 1500%, 2000%, 2500%, 3000%, or more, or any range in between, such as 5%-100%, higher or lower than that in a normal and/or a control sample.

In some embodiments, there is a change or a significant change in the heterogeneity of a lipid length of a ganglioside, if the amount or level of a ganglioside having a short lipid length (14-24 carbons) and a long lipid length (26-38 carbons) in a subject sample is at least or about 5%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 250%, 300%, 350%, 400%, 450%, 500%, 550%, 600%, 650%, 700%, 750%, 800%, 850%, 900%, 950%, 1000%, 1500%, 2000%, 2500%, 3000%, or more, or any range in between, such as 5%-100%, higher or lower than that in a normal and/or a control sample.

### Control

A control refers to any suitable reference standard, such as a normal patient, cultured primary cells/tissues isolated from a subject such as a normal subject, adjacent normal cells/tissues obtained from the same organ or body location of the patient, a tissue or cell sample isolated from a normal subject, or a primary cells/tissues obtained from a depository. In other embodiments, the control may comprise an expression level (e.g., the level of a ganglioside or one or more of its lipoforms) and/or lipid tail length of a ganglioside of a subject, such as a normal or healthy subject. In some embodiments, the control may be from a diseased subject, e.g., a subject afflicted with a cancer.

A control also refers to any reference standard suitable to provide a comparison to the expression products in the test sample. In certain embodiments, the control comprises obtaining a control sample from which the level of a ganglioside, one or more of its lipoforms, or the lipid length of a ganglioside is detected and compared to the same from the test sample. Such a control sample may comprise any suitable sample, including but not limited to a sample from a control cancer patient (can be stored sample or previous sample measurement) with a known outcome; normal tissue or cells isolated from a subject, such as a normal patient or the cancer patient, cultured primary cells/tissues isolated from a subject such as a normal subject or the cancer patient, adjacent normal cells/tissues obtained from the same organ or body location of the cancer patient, a tissue or cell sample isolated from a normal subject, or a primary cells/tissues obtained from a depository. In some embodiments, the control may comprise a reference standard expression product (e.g., ganglioside) level from any suitable source, including but not limited to an expression product level range from normal tissue (or other previously analyzed control sample), a previously determined expression product level range within a test sample from a group of patients, or a set of patients with a certain outcome (for example, survival for one, two, three, four years, etc.) or receiving a certain treatment (for example, standard of care cancer therapy). In some embodiments, the control comprises samples drawn or collected longitudinally at different times, to evaluate a change in the level of a ganglioside, one or more of its lipoforms, or the lipid length of a ganglioside over time. It will be understood by those of skill in the art that such control samples and reference standard expression product levels can be used in combination as controls in the methods of the present invention.

In some embodiments, the amount of a ganglioside or a lipoform thereof may be determined within a sample relative to, or as a ratio of, the amount of another ganglioside or a lipoform thereof in the same sample. In some embodiments, the control comprises a ratio transformation of expression product levels, including but not limited to determining a ratio of product levels of two gangliosides, a ratio of a lipoform of a ganglioside vs. total ganglioside, or the ratio of short lipid length vs. long lipid length of a ganglioside in the test sample and comparing it to any suitable ratio of the same in a reference standard; determining product levels of the two or more gangliosides or lipoforms thereof in the test sample and determining a difference in product levels in any suitable control; and determining product levels of the two or more gangliosides in the test sample, normalizing their level to the level of housekeeping gene products in the test sample, and comparing to any suitable control. In preferred embodiments, the control comprises a control sample which is of the same lineage and/or type as the test sample. In other embodiments, the control may comprise product levels grouped as percentiles within or based on a set of patient samples, such as all patients with cancer. In some embodiments, a control product level is established wherein higher or lower levels of product relative to, for instance, a particular percentile, are used as the basis for predicting outcome. In other preferred embodiments, a control product level is established using product levels from cancer control patients with a known outcome, and the product levels from the test sample are compared to the control product level as the basis for predicting outcome. As demonstrated by the data provided herein, the methods of the present invention are not limited to use of a specific cut-point in comparing the level of product in the test sample to the control.

In some embodiments, a pre-determined marker amount can be any suitable standard. For example, the pre-determined marker amount can be obtained from the same or a different human for whom a patient selection is being assessed. In some embodiments, the pre-determined marker amount can be obtained from a previous assessment of the same patient. In such a manner, the progress of the selection of the patient can be monitored over time. In addition, the control can be obtained from an assessment of another human or multiple humans, *e.g.*, selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group.

Accordingly, in preferred embodiments, a control comprises a sample (e.g., serum or tissues) from a normal healthy person without cancer. In yet other preferred embodiments, a control comprises a sample (e.g., serum or tissues) from a patient who is being evaluated (e.g., diagnosis or prognosis). For example, the control sample may comprise (i) a historical sample of the patient, or (ii) the sample obtained from the patient in longitudinal studies, e.g., pre-therapy or post-therapy (e.g., cancer therapy). The use of such control allows comparison of a biomarker present in the same patient over time (e.g., during the progression of cancer).

### Diagnostic Assays

The present invention provides, in part, methods, systems, and code for accurately classifying whether a biological sample comprises a ganglioside (or one or more of its lipoforms) and/or whether the levels of a ganglioside (or one or more of its lipoforms) are modulated (*e.g.*, upregulated or downregulated), thereby indicative of the state of a disorder of interest, such as cancer. In some embodiments, the present invention is useful for classifying a sample (*e.g.*, from a subject) as associated with or at risk for cancer or a subtype thereof, mediated by a ganglioside using a statistical algorithm and/or empirical data (*e.g.*, the presence, absence, level, or the lipid length of a ganglioside or its lipoforms).

An exemplary method for detecting the level of a ganglioside, and thus useful for classifying whether a sample is associated with a cancer or a clinical subtype thereof or different stages of a cancer involves obtaining a biological sample from a test subject and detecting a ganglioside, one or more of its lipoforms, or the lipid tail length of the ganglioside in the sample using a mass spectrometry-based method.

In certain instances, the statistical algorithm is a single learning statistical classifier system. For example, a single learning statistical classifier system can be used to classify a sample as a cancer sample based upon a prediction or probability value and the presence or level of ganglioside. The use of a single learning statistical classifier system typically classifies the sample as a cancer sample with a sensitivity, specificity, positive predictive value, negative predictive value, and/or overall accuracy of at least or about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Other suitable statistical algorithms are well-known to those of skill in the art. For example, learning statistical classifier systems include a machine learning algorithmic technique capable of adapting to complex data sets (*e.g.*, panel of markers of interest) and making decisions based upon such data sets. In some embodiments, a single learning statistical classifier system such as a classification tree (*e.g.*, random forest) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.*, decision/classification trees such as random forests, classification and regression trees (C&RT), boosted trees, etc.), Probably Approximately Correct (PAC) learning, connectionist learning (*e.g.*, neural networks (NN), artificial neural networks (ANN), neuro fuzzy networks (NFN), network structures, perceptrons such as multi-layer perceptrons, multi-layer feed-forward networks, applications of neural networks, Bayesian learning in belief networks, etc.), reinforcement learning (*e.g.*, passive learning in a known environment such as naive learning, adaptive dynamic learning, and temporal difference learning, passive learning in an unknown environment, active learning in an unknown environment, learning action-value functions, applications of reinforcement learning, etc.), and genetic algorithms and evolutionary programming. Other learning statistical classifier systems include support vector machines (*e.g.*, Kernel methods), multivariate adaptive regression splines (MARS), Levenberg-Marquardt algorithms, Gauss-Newton algorithms, mixtures of Gaussians, gradient descent algorithms, and learning vector quantization (LVQ). In certain embodiments, the method of the present invention further comprises sending the sample classification results to a clinician (a non-specialist, *e.g.,* primary care physician; and/or a specialist, *e.g.*, a histopathologist or an oncologist).

In some embodiments, the method of the present disclosure further provides a diagnosis in the form of a probability that the individual has a cancer. For example, the individual can have about a 0%, 5%, 10%, 15%, 20%, 25%, 30%, 35%, 40%, 45%, 50%, 55%, 60%, 65%, 70%, 75%, 80%, 85%, 90%, 95%, or greater probability of having the cancer. In yet another embodiment, the method of the present invention further provides a prognosis of the cancer in the individual. In some instances, the method of classifying a sample as a cancer sample may be further based on the symptoms (*e.g.*, clinical factors) of the individual from which the sample is obtained. The symptoms or group of symptoms can be, for example, lymphocyte count, white cell count, erythrocyte sedimentation rate, diarrhea, abdominal pain, bloating, pelvic pain, lower back pain, cramping, fever, anemia, weight loss, anxiety, depression, and combinations thereof. In some instances, the method of classifying a sample as a cancer sample may be further based on genetic mutations and/or predisposition to cancer, irrespective of the symptoms. In some embodiments, the diagnosis of an individual as having a cancer is followed by administering to the individual a therapeutically effective amount of a cancer therapy (*e.g*., chemotherapeutic agents). In some embodiments, the diagnosis of an individual as having a cancer is followed by treating the individual with a cancer therapy.

In some embodiments, the methods further involve obtaining a control biological sample (*e.g*., biological sample from a subject who does not have a cancer), a biological sample from the subject during remission or before developing a cancer, or a biological sample from the subject during treatment for developing a cancer.

In some embodiments, the methods comprise analyzing the control sample to detect a ganglioside, one or more of its lipoforms, or lipid length of a ganglioside, such that the presence and/or the level of said ganglioside, one or more of its lipoforms, or lipid length of a ganglioside, is detected in the biological sample, and comparing the presence or the level of a ganglioside in the control sample with the presence or the level of a ganglioside in the test sample.

A preferred biological sample is a serum, blood, saliva, tumor microenvironment, peritumoral cells/tissues, or intratumoral cells/tissues, isolated by conventional means from a subject. A person of ordinary skill in the art would understand that cell- or tissue samples may need further processing (e.g., homogenize and/or partially purify the lipid fraction; see Example 1).

The level and/or heterogeneity of a ganglioside, one or more of its lipoforms, or lipid length of a ganglioside, as determined by the methods of the present disclosure, correlate with different grades of a cancer. Accordingly, in some embodiments, the methods of the present disclosure can be used to determine a grade of a cancer, based on the level and/or heterogeneity of the ganglioside, one or more of its lipoforms, or lipid length of a ganglioside determined as described herein. A cancer's grade describes how abnormal the cancer cells and tissue look under a microscope when compared to healthy cells. Cancer cells that look and organize most like healthy cells and tissue are low grade tumors. Doctors describe these cancers as being well differentiated. Lower grade cancers are typically less aggressive and have a better prognosis. The more abnormal the cells look and organize themselves, the higher the cancer's grade. Cancer cells with a high grades tend to be more aggressive. They are called poorly differentiated or undifferentiated. Some cancers have their own system for grading tumors. Many others use a standard 1-4 grading scale.
- Grade 1: Tumor cells and tissue looks most like healthy cells and tissue. These are called well-differentiated tumors and are considered low grade.
- Grade 2: The cells and tissue are somewhat abnormal and are called moderately differentiated. These are intermediate grade tumors.
- Grade 3: Cancer cells and tissue look very abnormal. These cancers are considered poorly differentiated, since they no longer have an architectural structure or pattern. Grade 3 tumors are considered high grade.
- Grade 4: These undifferentiated cancers have the most abnormal looking cells. These are the highest grade and typically grow and spread faster than lower grade tumors.
As used herein, low grade cancer refers to Grade I cancer; and high grade cancer refers to cancer of Grades 2-4.

Similarly, the level and/or heterogeneity of a ganglioside, one or more of its lipoforms, or lipid length of a ganglioside, as determined by the methods of the present disclosure, correlate with different stages of a cancer. Accordingly, in some embodiments, the compositions and methods of the present disclosure can be used to determine a grade of a cancer, based on the level and/or heterogeneity of the ganglioside, one or more of its lipoforms, or lipid length of a ganglioside determined as described herein.

A cancer's stage explains how large the primary tumor is and how far the cancer has spread in the patient's body. There are several different staging systems. Many of these have been created for specific kinds of cancers. Others can be used to describe several types of cancer. One common system that many people are aware of puts cancer on a scale of 0 to IV.
- Stage 0 is for abnormal cells that haven't spread and are not considered cancer, though they could become cancerous in the future. This stage is also called "in-situ."
- Stage I through Stage III are for cancers that haven't spread beyond the primary tumor site or have only spread to nearby tissue. The higher the stage number, the larger the tumor and the more it has spread.
- Stage IV cancer has spread to distant areas of the body.
As used herein, cancer at the early/low stage refers to cancer at Stage I; and cancer at the late/high/advanced stage includes cancer at Stage II to Stage IV.

Likewise, the level and/or heterogeneity of a ganglioside, one or more of its lipoforms, or lipid length of a ganglioside, as determined by the methods of the present disclosure, correlate with the tumor burden. Accordingly, in some embodiments, the compositions and methods of the present disclosure can be used to determine the tumor burden of a subject, based on the level and/or heterogeneity of the lipid length of at least one ganglioside determined as described herein. Tumor burden (or tumor load) is defned as the total amount of tumor (cells/mass) distributed in the patients' body, including bone marrow. In Response Evaluation Criteria in Solid Tumors (RECIST) analysis, tumor burden is considered the sum of the longest diameters of all measurable lesions. Various methods can be used to determine the tumor burden in a subject. For example, computed tomography (CT) and magnetic resonance (MR) imaging have been used to assess tumor response based on morphologic (size, location) criteria, specifcally by using RECIST. RECIST classifcation describes lesions' size and distinguishes 4 types of treatment response - stable disease (SD), partial response (PR), complete response (CR) or progressive disease (PD).

### Prognostic Assays

The term "prognosis" includes a prediction of the probable course and outcome of cancer or the likelihood of recovery from the disease. In some embodiments, the use of statistical algorithms provides a prognosis of cancer in an individual. For example, the prognosis can be surgery, development of a clinical subtype of cancer (*e.g.*, solid tumors, such as lung cancer, melanoma, and renal cell carcinoma), development of one or more clinical factors, development of intestinal cancer, or recovery from the disease.

The assays described herein, such as the preceding diagnostic assays or the following assays, can be utilized to to determine whether a subject can be administered an agent (*e.g.*, an agonist, antagonist, peptidomimetic, polypeptide, peptide, nucleic acid, small molecule, immunotherapy, immune checkpoint inhibition therapy, or other drug candidate) to treat a cancer. For example, such methods can be used to determine whether a subject can be effectively treated with one or a combination of agents. Thus, the present disclosure provides methods for determining whether a subject can be effectively treated with one or more agents for treating a cancer in which a test sample is obtained and a ganglioside is detected.

Other aspects of the present disclosure include uses of the methods described herein for association and/or stratification analyses in which a ganglioside, one or more of its lipoforms, or lipid length of a ganglioside in biological samples from individuals with a cancer, are analyzed and the information is compared to that of controls (*e.g.*, individuals who do not have the cancer; controls may be also referred to as "healthy" or "normal" individuals or at early timepoints in a given time lapse study) who are preferably of similar age and race. The appropriate selection of patients and controls is important to the success of association and/or stratification studies. Therefore, a pool of individuals with well-characterized phenotypes is extremely desirable. Criteria for cancer diagnosis, cancer predisposition screening, predicting clinical outcomes, cancer prognosis, determining drug responsiveness (pharmacogenomics), drug toxicity screening, etc. are described herein.

Different study designs may be used for genetic association and/or stratification studies (Modern Epidemiology, Lippincott Williams & Wilkins (1998), 609-622). Observational studies are most frequently carried out in which the response of the patients is not interfered with. The first type of observational study identifies a sample of persons in whom the suspected cause of the disease is present and another sample of persons in whom the suspected cause is absent, and then the frequency of development of disease in the two samples is compared. These sampled populations are called cohorts, and the study is a prospective study. The other type of observational study is case-control or a retrospective study. In typical case-control studies, samples are collected from individuals with the phenotype of interest (cases) such as certain manifestations of a disease, and from individuals without the phenotype (controls) in a population (target population) that conclusions are to be drawn from. Then the possible causes of the disease are investigated retrospectively. As the time and costs of collecting samples in case-control studies are considerably less than those for prospective studies, case-control studies are the more commonly used study design in genetic association studies, at least during the exploration and discovery stage.

After all relevant phenotypic and/or genotypic information has been obtained, statistical analyses are carried out to determine if there is any significant correlation between the presence of an allele or a genotype with the phenotypic characteristics of an individual. Preferably, data inspection and cleaning are first performed before carrying out statistical tests for genetic association. Epidemiological and clinical data of the samples can be summarized by descriptive statistics with tables and graphs well-known in the art. Data validation is preferably performed to check for data completion, inconsistent entries, and outliers. Chi-squared tests and t-tests (Wilcoxon rank-sum tests if distributions are not normal) may then be used to check for significant differences between cases and controls for discrete and continuous variables, respectively.

An important decision in the performance of genetic association tests is the determination of the significance level at which significant association can be declared when the p-value of the tests reaches that level. In an exploratory analysis where positive hits will be followed up in subsequent confirmatory testing, an unadjusted p-value <0.2 (a significance level on the lenient side), for example, may be used for generating hypotheses for significant association of a ganglioside level with certain phenotypic characteristics of a cancer. It is preferred that a p-value <0.05 (a significance level traditionally used in the art) is achieved in order for the level to be considered to have an association with a cancer. When hits are followed up in confirmatory analyses in more samples of the same source or in different samples from different sources, adjustment for multiple testing will be performed as to avoid excess number of hits while maintaining the experiment-wise error rates at 0.05. While there are different methods to adjust for multiple testing to control for different kinds of error rates, a commonly used but rather conservative method is Bonferroni correction to control the experiment-wise or family-wise error rate (Multiple comparisons and multiple tests, Westfall et al, SAS Institute (1999)). Permutation tests to control for the false discovery rates, FDR, can be more powerful (Benjamini and Hochberg, Journal of the Royal Statistical Society, Series B 57, 1289-1300, 1995, Resampling-based Multiple Testing, Westfall and Young, Wiley (1993)). Such methods to control for multiplicity would be preferred when the tests are dependent and controlling for false discovery rates is sufficient as opposed to controlling for the experiment-wise error rates.

Once individual risk factors, genetic or non-genetic, have been found for the predisposition to disease, a classification/prediction scheme can be set up to predict the category (for instance, disease or no-disease) that an individual will be in depending on his phenotype and/or genotype and other non-genetic risk factors. Logistic regression for discrete trait and linear regression for continuous trait are standard techniques for such tasks (Applied Regression Analysis, Draper and Smith, Wiley (1998)). Moreover, other techniques can also be used for setting up classification. Such techniques include, but are not limited to, MART, CART, neural network, and discriminant analyses that are suitable for use in comparing the performance of different methods (The Elements of Statistical Learning, Hastie, Tibshirani & Friedman, Springer (2002)).

### Exemplary Embodiments

In certain aspects, a method of detecting the presence, level, and/or lipid length of at least one ganglioside and/or at least one lipoform of a ganglioside, the method comprising detecting said ganglioside and/or at least one lipoform of a ganglioside in a sample using mass spectrometry, optionally wherein the sample is from a subject having cancer, a subject suspected of having cancer, or a cancer-free subject.

Mass spectrometry may be any method of the present disclosure or any other mass spectrometry method known in the art.

In some embodiments, the mass spectrometry is selected from LC-MS, LC-MS/MS, LC-ESI-MS/MS, LC-ESI-CID-MS/MS, nanobore LC-ESI-MS, and nanobore LC-ESI-MS/MS.

In certain aspects, provided herein are diagnostic and prognostic methods. For example, in certain aspects, provided herein is a method of diagnosing a cancer in a subject, the method comprising: a) determining the level of at least one ganglioside and/or at least one lipoform of a ganglioside in a subject sample; and b) comparing said level of the at least one ganglioside and/or at least one lipoform of a ganglioside to that in a control sample, wherein a significantly higher level (at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000%) of the at least one ganglioside and/or at least one lipoform of a ganglioside in the subject sample as compared to the level in the control sample indicates that the subject has a cancer.

In certain aspects, provided herein is a method of identifying a subject having a cancer, the method comprising: a) determining the level of at least one ganglioside and/or at least one lipoform of a ganglioside in a subject sample; and b) comparing said level of the at least one ganglioside and/or at least one lipoform of a ganglioside to that in a control sample, wherein a significantly higher level (at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000%) of the at least one ganglioside and/or at least one lipoform of a ganglioside in the subject sample as compared to the level in the control sample identifies the subject as having a cancer.

In certain aspects, provided herein is a method of determining a stage of a cancer, the method comprising: a) determining the level of at least one ganglioside and/or at least one lipoform of a ganglioside in a subject sample; and b) comparing said level of the at least one ganglioside and/or at least one lipoform of a ganglioside to that in a control sample, wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside in the subject sample as compared to the level in the control sample indicates that the subject has an early-stage cancer; and/or wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside in the subject sample as compared to the level in the control sample indicates that the subject has a late-stage cancer.

In some embodiments, at least 100% and no more than 200% increase in the level of the at least one ganglioside and/or at least one lipoform of a ganglioside in the subject sample as compared to the level in the control sample indicates that the subject has an early-stage cancer. In some embodiments, at least 200% increase in the level of the at least one ganglioside and/or at least one lipoform of a ganglioside in the subject sample as compared to the level in the control sample indicates that the subject has a late-stage cancer.

In certain aspects, provided herein is a method of determining a grade of a cancer, the method comprising: a) determining the level of at least one ganglioside and/or at least one lipoform of a ganglioside in a subject sample; and b) comparing said level of the at least one ganglioside and/or at least one lipoform of a ganglioside to that in a control sample, wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a Grade I cancer; wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a Grade II cancer; wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a Grade III cancer; and/or wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a Grade IV cancer.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a low grade cancer (e.g., Grade I).

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has an high grade cancer (e.g., Grade II, III, or IV).

In some embodiments, at least 100% and no more than 200% increase in the level of the at least one ganglioside and/or at least one lipoform of a ganglioside in the subject sample as compared to the level in the control sample indicates that the subject has a low grade cancer (e.g., Grade I). In some embodiments, at least 200% increase in the level of the at least one ganglioside and/or at least one lipoform of a ganglioside in the subject sample as compared to the level in the control sample indicates that the subject has a high-grade cancer (e.g., Grade II, III, or IV).

In certain aspects, provided herein is a method of determining a tumor burden of a cancer, the method comprising: a) determining the level of at least one ganglioside and/or at least one lipoform of a ganglioside in a subject sample; and b) comparing said level of the at least one ganglioside and/or at least one lipoform of a ganglioside to that in a control sample, wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a low tumor burden; and/or wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase in the level of the at least one ganglioside and/or at least one lipoform of a ganglioside in the subject sample as compared to the level in the control sample indicates that the subject has a high tumor burden.

In some embodiments, at least 100% and no more than 200% increase in the level of the at least one ganglioside and/or at least one lipoform of a ganglioside in the subject sample as compared to the level in the control sample indicates that the subject has a low tumor burden. In some embodiments, at least 200% increase in the level of the at least one ganglioside and/or at least one lipoform of a ganglioside in the subject sample as compared to the level in the control sample indicates that the subject has a high tumor burden.

In certain aspects, provided herein is a method of detecting a recurrence of a cancer in a subject, the method comprising: a) obtaining or providing a sample from the subject whose cancer has regressed after receiving cancer treatment; b) determining the level of at least one ganglioside and/or at least one lipoform of a ganglioside in the subject sample; and c) comparing said level of the at least one ganglioside and/or at least one lipoform of a ganglioside to that in a control sample, wherein a significantly higher level of the at least one ganglioside and/or at least one lipoform of a ganglioside in the subject sample as compared to the level in the control sample indicates a recurrence of a cancer in the subject.

In certain aspects, provided herein is a method of detecting a minimal residual disease in a subject, the method comprising: a) obtaining or providing a sample from the subject in remission; b) determining the level of at least one ganglioside and/or at least one lipoform of a ganglioside in the subject sample; and c) comparing said level of the at least one ganglioside and/or at least one lipoform of a ganglioside to that in a control sample, wherein a significantly higher level of the at least one ganglioside and/or at least one lipoform of a ganglioside in the subject sample as compared to the level in the control sample indicates that the subject has a minimal residual disease.

In certain aspects, provided herein is a method of stratifying a subject afflicted with a cancer according to benefit from a cancer therapy (e.g., immunotherapy), the method comprising: a) determining the level of at least one ganglioside and/or at least one lipoform of a ganglioside in a sample from a subject administered with a cancer therapy; b) determining the level of the at least one ganglioside and/or at least one lipoform of a ganglioside in a control; and c) comparing the level of the at least one ganglioside and/or at least one lipoform of a ganglioside detected in steps a) and b); wherein no significant change or a decrease in the level of the at least one ganglioside and/or at least one lipoform of a ganglioside in the subject sample as compared to the level in the control is an indication that the subject afflicted with the cancer would benefit from the cancer therapy.In certain aspects, provided herein is a method of determining whether a subject afflicted with a cancer would likely respond or alternatively would likely not respond to a cancer therapy (e.g., immunotherapy), the method comprising: a) determining the level of at least one ganglioside and/or at least one lipoform of a ganglioside in a sample from a subject administered with a cancer therapy; b) determining the level of the at least one ganglioside and/or at least one lipoform of a ganglioside in a control; and c) comparing the level of the at least one ganglioside and/or at least one lipoform of a ganglioside detected in steps a) and b); wherein a significantly higher level of the at least one ganglioside and/or at least one lipoform of a ganglioside in the subject sample as compared to the level in the control is an indication that the subject afflicted with the cancer would not respond to the cancer therapy; and/or wherein no significant change or a decrease in the level of the at least one ganglioside and/or at least one lipoform of a ganglioside in the subject sample as compared to the level in the control is an indication that the subject afflicted with the cancer would respond to the cancer therapy.

In certain aspects, provided herein is a method for predicting the clinical outcome of a subject afflicted with a cancer, the method comprising: a) determining the level of at least one ganglioside and/or at least one lipoform of a ganglioside in a subject sample; b) determining the level of the at least one ganglioside and/or at least one lipoform of a ganglioside in a control; and c) comparing the level of the at least one ganglioside and/or at least one lipoform of a ganglioside determined in steps a) and b); wherein a significantly higher level of the at least one ganglioside and/or at least one lipoform of a ganglioside in the subject sample as compared to the level in the control is an indication that the subject has a poor clinical outcome.

In certain aspects, provided herein is a method of monitoring the progression of a cancer in a subject, the method comprising: a) detecting in a subject sample at a first point in time the level of at least one ganglioside and/or at least one lipoform of a ganglioside; b) repeating step a) at a subsequent point in time; and c) comparing the level of the at least one ganglioside and/or at least one lipoform of a ganglioside detected in steps a) and b) to monitor the progression of the cancer in the subject. In some embodiments, a significantly higher level of the at least one ganglioside and/or the at least one lipoform of a ganglioside in the subject sample relative to the control indicates a progression of the cancer (e.g., an increase in the tumor burden, an increase in the cancer stage, developing cancer by a subject at risk).

In some embodiments, the method monitors the progression of a cancer in a subject who has received a cancer therapy between the first point in time and the subsequent point in time. In some embodiments, the subject is at risk for developing a cancer.

In certain aspects, provided herein is a method of assessing the efficacy of a cancer therapy in a subject afflicted with a cancer, the method comprising: a) determining the level of at least one ganglioside and/or at least one lipoform of a ganglioside in a first sample obtained from a subject; b) repeating step a) during at least one subsequent point in time after administration of the cancer therapy; and c) comparing the level of at least one ganglioside and/or at least one lipoform of a ganglioside detected in steps a) and b), wherein a significantly lower level of the at least one ganglioside and/or at least one lipoform of a ganglioside in the at least one subsequent sample, relative to the first sample, is an indication that the therapy is efficacious to treat a cancer in the subject.

In some embodiments, the first and/or at least one subsequent sample is a portion of a single sample or pooled samples obtained from the subject.

In some embodiments, the cancer therapy is a surgery, chemotherapy, cancer vaccines, chimeric antigen receptors, radiation therapy, immunotherapy, a modulator of expression of immune checkpoint inhibitory proteins or ligands, or any combination thereof. In some embodiments, the immunotherapy is an immune checkpoint inhibition therapy. In some embodiments, the cancer therapy is avelumab, durvalumab, atezolizumab, BRAF/MEK inhibitor, a tyrosine kinase inhibitor, pembrolizumab, nivolumab, ipilimumab, or a combination thereof.

Numerous embodiments are further provided that can be applied to any aspect of the present invention described herein.

For example, the diagnostic and prognostic methods described herein may use any method known in the art to detect and determine the level of at least one ganglioside and/or at least one lipoform of a ganglioside.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% higher level of the at least one ganglioside and/or at least one lipoform of a ganglioside indicates a significantly higher level.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% lower level of the at least one ganglioside and/or at least one lipoform of a ganglioside indicates a significantly lower level.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase or decrease in the level of at least one ganglioside and/or at least one lipoform of a ganglioside indicates no significant change in the level of the at least one ganglioside.

Further provided herein are diagnostic and prognostic methods that use the heterogeneity or homogeneity of the lipid length (e.g., as determined using mass spectrometry) of at least one ganglioside. In some embodiments, a significant change in heterogeneity of the lipid length of the at least one ganglioside in the subject sample as compared to the control sample indicates that the subject has a cancer. Similarly, in some embodiments, a significant change in the heterogeneity of the lipid length of the at least one ganglioside in the subject sample as compared to the control sample identifies the subject as having a cancer.

Accordingly, in certain aspects, provided herein is a method of diagnosing a cancer in a subject, the method comprising: a) determining the lipid length of at least one ganglioside in a subject sample (e.g., using mass spectrometry); and b) comparing the said lipid length of the at least one ganglioside to that in a control sample, wherein a significant change in heterogeneity of the lipid length of the at least one ganglioside in the subject sample as compared to the control sample indicates that the subject has a cancer.In certain aspects, provided herein is a method of determining a stage of a cancer, the method comprising: a) determining the lipid length of at least one ganglioside in the subject sample using mass spectrometry; and b) comparing said lipid length of the at least one ganglioside to that in a control sample, wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change in the heterogeneity of the lipid length of the at least one ganglioside in the subject sample as compared to that in the control sample indicates that the subject has an early-stage cancer; and/or at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change in the heterogeneity of the lipid length of the at least one ganglioside in the subject sample as compared to that in the control sample indicates that the subject has a late-stage cancer.

In some embodiments, at least 100% and no more than 200% change (e.g., increase or decrease) in heterogeneity of the lipid length of the at least one ganglioside in the subject sample as compared to that in the control sample indicates that the subject has an early-stage cancer. In some embodiments, at least 200% change (e.g., increase or decrease) in heterogeneity of the lipid length of the at least one ganglioside in the subject sample as compared to that in the control sample indicates that the subject has a late-stage cancer.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase or decrease in (a) the level of a ganglioside having a short lipid length with respect to the level of a ganglioside having a long lipid length; or (b) the level of a ganglioside having a long lipid length with respect to the level of a ganglioside having a short lipid length; indicates that the subject has an early-stage or a late-stage cancer.

In certain aspects, provided herein is a method of determining a grade of a cancer, the method comprising: a) determining the lipid length of at least one ganglioside in the subject sample using mass spectrometry; and b) comparing the said lipid length of the at least one ganglioside to that in a control sample, wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change (e.g., increase or decrease) in heterogeneity of the lipid length of the at least one ganglioside in the subject sample as compared to that in the control sample indicates that the subject has a Grade I, Grade II, Grade III, or Grade IV cancer.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change (e.g., increase or decrease) in heterogeneity of the lipid length of the at least one ganglioside in the subject sample as compared to that in the control sample indicates that the subject has a low grade cancer (e.g., Grade I) or a high grade cancer (e.g., Grade II, III, or IV).

In some embodiments, at least 100% and no more than 200% change (e.g., increase or decrease) in heterogeneity of the lipid length of the at least one ganglioside in the subject sample as compared to that in the control sample indicates that the subject has a low grade cancer. In some embodiments, at least 200% change (e.g., increase or decrease) in heterogeneity of the lipid length of the at least one ganglioside in the subject sample as compared to that in the control sample indicates that the subject has a high grade cancer.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase or decrease in (a) the level of a ganglioside having a short lipid length with respect to the level of a ganglioside having a long lipid length; or (b) the level of a ganglioside having a long lipid length with respect to the level of a ganglioside having a short lipid length; indicates that the subject has a low grade or a high grade cancer.

In certain aspects, provided herein is a method of determining a tumor burden of a cancer, the method comprising: a) determining the lipid length of at least one ganglioside in the subject sample using mass spectrometry; and b) comparing said lipid length of the at least one ganglioside to that in a control sample, wherein at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change in the heterogeneity of the lipid length of the at least one ganglioside in the subject sample as compared to that in the control sample indicates that the subject has low tumor burden; and/or at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change in the heterogeneity of the lipid length of the at least one ganglioside in the subject sample as compared to that in the control sample indicates that the subject has a high tumor burden.

In some embodiments, at least 100% and no more than 200% change (e.g., increase or decrease) in heterogeneity of the lipid length of the at least one ganglioside in the subject sample as compared to that in the control sample indicates that the subject has a low tumor burden. In some embodiments, at least 200% change (e.g., increase or decrease) in heterogeneity of the lipid length of the at least one ganglioside in the subject sample as compared to that in the control sample indicates that the subject has a high tumor burden.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase or decrease in (a) the level of a ganglioside having a short lipid length with respect to the level of a ganglioside having a long lipid length; or (b) the level of a ganglioside having a long lipid length with respect to the level of a ganglioside having a short lipid length; indicates that the subject has low tumor burden or a high tumor burden.

In certain aspects, provided is a method of detecting a recurrence of a cancer in a subject, the method comprising: a) obtaining or providing a sample from the subject whose cancer has regressed after receiving cancer treatment; b) determining the lipid length of at least one ganglioside in the subject sample using mass spectrometry; and c) comparing the said lipid length of the at least one ganglioside to that in a control sample, wherein a significant change in heterogeneity of the lipid length of the at least one ganglioside in the subject sample as compared to that in the control sample indicates a recurrence of a cancer in the subject.

In certain aspects, provided herein is a method of detecting a minimal residual disease in a subject, the method comprising: a) obtaining or providing a sample from the subject in remission; b) determining the lipid length of at least one ganglioside in the subject sample using mass spectrometry; and c) comparing the said lipid length of the at least one ganglioside to that in a control sample, wherein a significant change in heterogeneity of the lipid length of the at least one ganglioside in the subject sample as compared to that in the control sample indicates that the subject has a minimal residual disease.

In certain aspects, provided herein is a method of stratifying subjects afflicted with a cancer according to benefit from a cancer therapy (e.g., immunotherapy), the method comprising: a) determining the lipid length of at least one ganglioside in a sample from a subject administered with a cancer therapy using mass spectrometry; b) determining the lipid length of the at least one ganglioside in a control; and c) comparing the lipid length of the at least one ganglioside detected in steps a) and b); wherein no significant change in heterogeneity of the lipid length of the at least one ganglioside in the subject sample as compared to that in the control is an indication that the subject afflicted with the cancer would benefit from the cancer therapy.

In certain aspects, provided herein is a method of determining whether a subject afflicted with a cancer would likely respond to a cancer therapy, the method comprising: a) determining the lipid length of at least one ganglioside in a sample from a subject administered with a cancer therapy using mass spectrometry; b) determining the lipid length of the at least one ganglioside in a control; and c) comparing the lipid length of the at least one ganglioside detected in steps a) and b); wherein a significant change in heterogeneity of the lipid length of the at least one ganglioside in the subject sample as compared to that in the control is an indication that the subject afflicted with the cancer would not respond to the cancer therapy; and/or wherein no significant change in heterogeneity of the lipid length of the at least one ganglioside in the subject sample as compared to that in the control is an indication that the subject afflicted with the cancer would respond to the cancer therapy.

In certain aspects, provided herein is a method for predicting the clinical outcome of a subject afflicted with a cancer, the method comprising: a) determining the lipid length of at least one ganglioside in a subject sample using mass spectrometry; b) determining the lipid length of the at least one ganglioside in a control; and c) comparing the lipid length of the at least one ganglioside determined in steps a) and b); wherein a significant change in heterogeneity of the lipid length of the at least one ganglioside in the subject sample as compared to the control sample is an indication that the subject has a poor clinical outcome.

In certain aspects, provided herein is a method of monitoring the progression of a cancer in a subject, the method comprising: a) detecting in a subject sample at a first point in time the lipid length of at least one ganglioside using mass spectrometry; b) repeating step a) at a subsequent point in time; and c) comparing heterogeneity of the lipid length of the at least one ganglioside detected in steps a) and b) to monitor the progression of the cancer in the subject, optionally wherein the subject is at risk for developing a cancer. In some embodiments, a significant change in heterogeneity of the lipid length of the at least one ganglioside in the subject sample relative to the control indicates a progression of the cancer (e.g., an increase in the tumor burden, an increase in the cancer stage, developing cancer by a subject at risk).

In some embodiments, between the first point in time and the subsequent point in time, the subject has received a cancer therapy.

In certain aspects, provided herein is a method of assessing the efficacy of a cancer therapy in a subject afflicted with a cancer, the method comprising: a) determining the lipid length of at least one ganglioside using mass spectrometry in a first sample obtained from a subject; b) repeating step a) during at least one subsequent point in time after administration of the cancer therapy; and c) comparing the level of at least one ganglioside detected in steps a) and b), wherein a significant change in heterogeneity of the lipid length of the at least one ganglioside in the second sample, relative to the first sample, is an indication that the therapy is efficacious to treat a cancer in the subject.

In some embodiments, the first and/or at least one subsequent sample is a portion of a single sample or pooled samples obtained from the subject.

The methods of the present disclosure (e.g., diagnostic and prognostic methods described herein) may use any method known in the art to detect and determine the heterogeneity of the lipid length of the at least one ganglioside. In preferred embodiments, the heterogeneity of the lipid length of the at least one ganglioside is determined by mass spectrometry (e.g., LC-MS, LC-MS/MS, LC-ESI-MS/MS, LC-ESI-CID-MS/MS, nanobore LC-ESI-MS, nanobore LC-ESI-MS/MS, or any other mass spectrometry methods of the present disclosure or those known in the art).

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change in the heterogeneity or homogeneity of the lipid length of the at least one ganglioside indicates a significant change in heterogeneity or homogeneity.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase or decrease in (a) the level of a ganglioside having a short lipid length with respect to the level of a ganglioside having a long lipid length; or (b) the level of a ganglioside having a long lipid length with respect to the level of a ganglioside having a short lipid length; indicates a significant change in the heterogeneity or homogeneity of the lipid length.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% change in the heterogeneity or homogeneity of the lipid length of the at least one ganglioside indicates no significant change in heterogeneity or homogeneity.

In some embodiments, at least, about, or no more than 10%, 20%, 30%, 40%, 50%, 60%, 70%, 80%, 90%, 100%, 110%, 120%, 130%, 140%, 150%, 160%, 170%, 180%, 190%, 200%, 210%, 220%, 230%, 240%, 250%, 260%, 270%, 280%, 290%, 300%, 310%, 320%, 330%, 340%, 350%, 360%, 370%, 380%, 390%, 400%, 410%, 420%, 430%, 440%, 450%, 460%, 470%, 480%, 490%, 500%, 510%, 520%, 530%, 540%, 550%, 560%, 570%, 580%, 590%, 600%, 610%, 620%, 630%, 640%, 650%, 660%, 670%, 680%, 690%, 700%, 710%, 720%, 730%, 740%, 750%, 760%, 770%, 780%, 790%, 800%, 810%, 820%, 830%, 840%, 850%, 860%, 870%, 880%, 890%, 900%, 910%, 920%, 930%, 940%, 950%, 960%, 970%, 980%, 990%, 1000% increase or decrease in (a) the level of a ganglioside having a short lipid length with respect to the level of a ganglioside having a long lipid length; or (b) the level of a ganglioside having a long lipid length with respect to the level of a ganglioside having a short lipid length; indicates no significant change in the heterogeneity or homogeneity of the lipid length.

Numerous embodiments are further provided that can be applied to any aspect of the present invention described herein. For example, in some embodiments, the cancer therapy is a surgery, chemotherapy, cancer vaccines, chimeric antigen receptors, radiation therapy, immunotherapy, a modulator of expression of immune checkpoint inhibitory proteins or ligands, or any combination thereof. In some embodiments, the immunotherapy is an immune checkpoint inhibition therapy. In some embodiments, the cancer therapy is avelumab, durvalumab, atezolizumab, BRAF/MEK inhibitor, a tyrosine kinase inhibitor, pembrolizumab, nivolumab, ipilimumab, or a combination thereof.

In some embodiments, the at least one ganglioside and/or at least one lipoform of a ganglioside is a tumor-associated ganglioside and/or a lipoform of a tumor-associated ganglioside. In some embodiments, the tumor-associated ganglioside is selected from GD2, GD3, GD1b, GT1b, fucosyl-GM1, GloboH, polysialic acid (PSA), GM2, GM3, sialyl-Lewis^{X}, sialyl-Lewis^{Y}, sialyl-Lewis^{A}, sialyl-Lewis^{B}, Lewis^{Y}, any portion thereof, any lipoform or isoform thereof, and any combination of two or more thereof.

In preferred embodiments, the at least one ganglioside and/or at least one lipoform of a ganglioside comprises GM2, GD3, GD2, GD1b, any lipoform thereof, or any combination thereof.

In some embodiments, the at least one ganglioside and/or at least one lipoform of a ganglioside comprises a combination of two or more selected from GM2, GD3, GD2, GD1b, and any lipoform thereof.

In some embodiments, the at least one ganglioside and/or the at least one lipoform of a ganglioside comprises GD3(d18:1/16:0), GD3(d18:1/23:0), GD3(d18:1/24:1), GD2(d18:1/16:0), GD1b(d18:1/16:0), GD1b(d18:1/24:1), GD1b(d18:1/18:1), or any combination of two or more thereof.

In some embodiments, the at least one ganglioside and/or the at least one lipoform of a ganglioside is selected from GD3(d18:1/16:0), GD3(d18:1/23:0), GD3(d18:1/24:1), GD2(d18:1/16:0), GD1b(d18:1/16:0), GD1b(d18:1/24:1), GD1b(d18:1/18:1), and any combination of two or more thereof.

In some embodiments, the at least one ganglioside and/or the at least one lipoform of a ganglioside comprises GD3(d18:1/23:0) and/or GD3(d18:1/24:1).

In some embodiments, the at least one ganglioside and/or the at least one lipoform of a ganglioside is GD3(d18:1/23:0) and/or GD3(d18:1/24:1).

In some embodiments, the at least one ganglioside and/or the at least one lipoform of a ganglioside comprises GD3(d18:1/23:0).

In some embodiments, the at least one ganglioside and/or the at least one lipoform of a ganglioside is GD3(d18:1/23:0).

In some embodiments, the at least one ganglioside and/or the at least one lipoform of a ganglioside comprises an acyl chain 24:1.

In some embodiments, the acyl chain 24:1 is present in GD3 and/or GD1b.

In some embodiments, the cancer is selected from the group consisting of neuroblastoma, lymphoma, leukemia, melanoma, glioma, small cell lung cancer, breast carcinoma, ovarian cancer, soft tissue sarcomas, osteosarcoma, Ewing's sarcoma, desmoplastic round cell tumor, rhabdomyosarcoma, retinoblastoma, non-small cell lung cancer, renal cell cancer, Wilms tumor, prostate cancer, gastric cancer, endometrial cancer, pancreatic cancer, and colon cancer.

In some embodiments, the cancer is selected from the group consisting of neuroblastoma, lymphoma, leukemia, melanoma, glioma, small cell lung cancer, breast carcinoma, ovarian cancer, soft tissue sarcomas, osteosarcoma, Ewing's sarcoma, desmoplastic round cell tumor, rhabdomyosarcoma, retinoblastoma.

In some embodiments, the cancer is ovarian cancer, melanoma, renal cancer, or lung cancer.

In preferred embodiments, the cancer is ovarian cancer.

In some embodiments, the cancer is a borderline tumor.

In some embodiments, the sample comprises cells, serum, blood, peritumoral tissue, and/or intratumoral tissue obtained from the subject (e.g., biopsy). In preferred embodiments, the sample comprises liquid biopsy (comprising liquid). Accordingly, in some embodiments, the sample comprises serum or blood.

In some embodiments, a significantly higher level of at least one ganglioside and/or at least one lipoform of a ganglioside comprises an at least twenty percent increase of the level of the at least one ganglioside and/or at least one lipoform of a ganglioside.

In some embodiments, a significantly lower level of at least one ganglioside and/or at least one lipoform of a ganglioside comprises an at least twenty percent decrease of the level of the at least one ganglioside and/or at least one lipoform of a ganglioside.

In preferred embodiments, a significantly higher or lower level of at least one ganglioside and/or at least one lipoform of a ganglioside is the level that is at least or about 50% higher or lower than the level of control (e.g., a non-cancerous sample). In other embodiments, a significantly higher or lower level of at least one ganglioside and/or at least one lipoform of a ganglioside is the level that is at least or about 25% higher or lower than the level of the previous reading of the subject in a longitudinal study.

In some embodiments, a significant change in the heterogeneity of lipid length of at least one ganglioside comprises an at least twenty percent change (e.g., increase or decrease) in the subject sample relative to the control sample.

In some embodiments, the control sample is a sample from a cancer-free subject.

In other embodimnets, the control sample is a sample from a subject afflicted with a cancer.

In some embodiments, the control sample is a sample from the subject (e.g., first sample collected from the subject in longitudinal collections, to evaluate changes over time of the level of the at least one ganglioside).

In preferred embodiments, the diagnostic and/or prognostic methods described herein further comprise treating the subject with a cancer therapy of the present disclosure or a cancer therapy known in the art. For example, a subject determined to be in need of treatment based on the results of diagnostic and/or prognostic methods of the present disclosure may be treated with a cancer therapy. In some embodiments, the diagnostic and/or prognostic methods further comprise recommending, prescribing, and/or administering to the subject a cancer therapy (e.g., immune checkpoint inhibition therapy) of the present disclosure or a cancer therapy known in the art.

In some embodiments, the subject is afflicted with a cancer. In some such embodiments, the subject is asymptomatic.

In some embodiments, the subject is a mammal (e.g., humans, pets (e.g., dogs, cats), livestock).

In some embodiments, the subject is an animal model of cancer, a dog, a cat, or a human.

In preferred embodiments, the subject is a human.

### Treating Patients & Monitoring of Effects During Clinical Trials

Methods of the present disclosure can be followed by treating the subject whose sample was tested in said methods. For example, a subject who was diagnosed with cancer using a method described herein can be treated with cancer therapy (e.g., the standard of care or cancer therapy described herein or those known in the art).

Monitoring the influence of agents (*e.g.*, compounds, drugs or small molecules, immunotherapy, cancer therapy described herein or those known in the art) on the level of a ganglioside and/or at least one lipoform of a ganglioside can be applied not only in basic drug screening, but also in clinical trials. For example, the effectiveness of an agent determined by a screening assay as described herein to decrease the level of a ganglioside can be monitored in clinical trials of subjects, detectable by the mass spectrometry-based methods. In such clinical trials, the level of a ganglioside and/or symptoms or other markers of the cancer, can be used as a "read out" or marker of the phenotype of a particular cell, tissue, or system.

In preferred embodiments, the present disclosure provides a method for monitoring the effectiveness of treatment of a subject with an agent (*e.g.*, an agonist, antagonist, peptidomimetic, polypeptide, peptide, nucleic acid, small molecule, immunotherapy, immune checkpoint inhibition therapy, or other drug candidate) including the steps of (i) obtaining a pre-administration sample from a subject prior to administration of the agent; (ii) detecting the level of at least one ganglioside and/or at least one lipoform of a ganglioside in the preadministration sample; (iii) obtaining one or more post-administration samples from the subject; (iv) detecting the level of the at least one ganglioside and/or at least one lipoform of a ganglioside in the post-administration samples; (v) comparing the level of the at least one ganglioside and/or at least one lipoform of a ganglioside in the pre-administration sample with the at least one ganglioside and/or at least one lipoform of a ganglioside in the post administration sample or samples; and (vi) altering the administration of the agent to the subject accordingly. For example, increased administration of the agent may be desirable to decrease the level of at least one ganglioside and/or at least one lipoform of a ganglioside to lower levels than detected, *i.e.*, to increase the effectiveness of the agent. According to such an embodiment, at least one ganglioside and/or at least one lipoform of a ganglioside may be used as an indicator of the effectiveness of an agent, even in the absence of an observable phenotypic response. Similarly, at least one ganglioside and/or at least one lipoform of a ganglioside analysis, such as by a mass spectrometry-based methods, can also be used to select patients who will receive a cancer therapy (e.g., immunotherapy, immune checkpoint inhibition therapy).

### Sample

Biological samples can be collected from a variety of sources from a subject including a body fluid sample, cell sample, or a tissue sample. Body fluids refer to fluids that are excreted or secreted from the body as well as fluids that are normally not (*e.g.*, amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit). In some embodiments, the subject and/or control sample is selected from the group consisting of cells, cell lines, whole blood, serum, plasma, buccal scrape, saliva, cerebrospinal fluid, and bone marrow. In some embodiments, the samples can contain live cells/tissue, fresh frozen cells, fresh tissue, biopsies, fixed cells/tissue, cells/tissue embedded in a medium, such as paraffin, histological slides, or any combination thereof. In some embodiments, the samples can contain live cells/tissue, fresh frozen cells, fresh tissue, and/or biopsies.

The samples can be collected from individuals repeatedly over a longitudinal period of time (*e.g.*, once or more on the order of days, weeks, months, annually, biannually, etc.).

Sample preparation and separation can involve any of the procedures, depending on the type of sample collected and/or analysis of biomarker measurement(s). Such procedures include, by way of example only, concentration, dilution, adjustment of pH, removal of high abundance polypeptides (*e.g.*, albumin, gamma globulin, and transferrin, etc.), addition of preservatives and calibrants, addition of protease inhibitors, addition of denaturants, desalting of samples, concentration of sample proteins, extraction and purification of lipids. In some embodiments, certain cell types are purified based on at least one marker present on the cell surface.

A sample may comprise a fixed molecule. A molecule is "fixed" or "affixed" to a substrate if it is covalently or non-covalently associated with the substrate such the substrate can be rinsed with a fluid (*e.g.* standard saline citrate, pH 7.4) without a substantial fraction of the molecule dissociating from the substrate.

As described herein, in some embodiments, the level of at least one ganglioside and/or at least one lipoform of a ganglioside measurement(s) in a sample from a subject is compared to a control biological sample (*e.g.*, biological sample from a subject who does not have a cancer), a control biological sample from the subject during remission or before developing a cancer, or a control biological sample from the subject during treatment for developing a cancer. In some embodiments, a control biological sample is from a subject prior to treatment with a certain therapy. In some embodiments, wherein a subject is treated with multiple rounds of one or more therapies, a control biological sample may be from an earlier or later time point with respect to the subject sample during such treatment. For example, a subject sample after third rounds of therapy may be compared with a control subject sample after the first round of therapy.

In some embodiments, the level of at least one ganglioside and/or at least one lipoform of a ganglioside measurement(s) in a sample from a subject is compared to a predetermined control (standard) sample. The sample from the subject is typically from a diseased tissue, such as cancer cells or tissues. The control sample can be from the same subject or from a different subject. The control sample is typically a normal, non-diseased sample. However, in some embodiments, such as for staging of disease or for evaluating the efficacy of treatment, the control sample can be from a diseased tissue. The control sample can be a combination of samples from several different subjects. In some embodiments, the biomarker amount and/or activity measurement(s) from a subject is compared to a pre-determined level. This pre-determined level is typically obtained from normal samples.

As described herein, a "pre-determined" biomarker amount measurement(s) may be a biomarker amount measurement(s) used to, by way of example only, evaluate a subject that may be selected for treatment, evaluate a response to a cancer therapy, and/or evaluate a response to a combination of anti-cancer therapies. A pre-determined biomarker amount and/or activity measurement(s) may be determined in populations of patients with or without cancer. The pre-determined biomarker amount measurement(s) can be a single number, equally applicable to every patient, or the pre-determined biomarker amount measurement(s) can vary according to specific subpopulations of patients. Age, weight, height, and other factors of a subject may affect the pre-determined biomarker amount measurement(s) of the individual. Furthermore, the pre-determined biomarker amount can be determined for each subject individually. In some embodiments, the amounts determined and/or compared in a method described herein are based on absolute measurements.

In some embodiments, the amounts determined and/or compared in a method described herein are based on relative measurements, such as ratios (*e.g.*, biomarker level before a treatment vs. after a treatment, such biomarker measurements relative to a spiked or man-made control, such biomarker measurements relative to the expression of a housekeeping gene, and the like). For example, the relative analysis can be based on the ratio of pre-treatment biomarker measurement as compared to post-treatment biomarker measurement. Pre-treatment biomarker measurement can be made at any time prior to initiation of anti-cancer therapy. Post-treatment biomarker measurement can be made at any time after initiation of anti-cancer therapy. In some embodiments, post-treatment biomarker measurements are made 1, 2, 3, 4, 5, 6, 7, 8, 9, 10, 11, 12, 13, 14, 15, 16, 17, 18, 19, 20 weeks or more after initiation of anti-cancer therapy, and even longer toward indefinitely for continued monitoring. Treatment can comprise one or more anti-cancer therapies, e.g., immune checkpoint inhibitors.

The pre-determined biomarker amount measurement(s) can be any suitable standard. For example, the pre-determined biomarker amount measurement(s) can be obtained from the same or a different human for whom a patient selection is being assessed. In some embodiments, the pre-determined biomarker amount measurement(s) can be obtained from a previous assessment of the same patient. In such a manner, the progress of the selection of the patient can be monitored over time. In addition, the control can be obtained from an assessment of another human or multiple humans, *e.g.*, selected groups of humans, if the subject is a human. In such a manner, the extent of the selection of the human for whom selection is being assessed can be compared to suitable other humans, *e.g.*, other humans who are in a similar situation to the human of interest, such as those suffering from similar or the same condition(s) and/or of the same ethnic group.

In some embodiments of the present disclosure the change of biomarker amount measurement(s) from the pre-determined level is about 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0 fold or greater, or any range in between, inclusive. Such cutoff values apply equally when the measurement is based on relative changes, such as based on the ratio of pre-treatment biomarker measurement as compared to post-treatment biomarker measurement.

### Cancer

Cancer, tumor, or hyperproliferative disorder refer to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within an animal, or may be a non-tumorigenic cancer cell, such as a leukemia cell. Cancers include, but are not limited to, B cell cancer, *e.g.,* multiple myeloma, Waldenström's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematologic tissues, and the like. Other non-limiting examples of types of cancers applicable to the methods encompassed by the present invention include human sarcomas and carcinomas, *e.g.*, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma (SCLC), bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma; leukemias, *e.g.*, acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, cancers are epithlelial in nature and include but are not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (*e.g.*, serous ovarian carcinoma), or breast carcinoma. The epithelial cancers may be characterized in various other ways including, but not limited to, serous, endometrioid, mucinous, clear cell, Brenner, or undifferentiated.

The compositions and methods of the present inventions may be used to detect various cancers, including ovarian cancer, small cell lung cancer (SCLC), or melanoma. Notably, the compositions and methods of the present inventions are particularly useful in detecting borderline tumors. Accordingly, the present inventions can be used to diagnose or prognose borderline tumors, including but not limited to, borderline ovarian tumors.

### Borderline tumors

Borderline tumors are a heterogeneous group of lesions defined histologically by atypical epithelial proliferation without stromal invasion.

Similarly, borderline tumors of the ovary (also called tumors of low malignant potential) are a heterogeneous group of lesions defined histologically by atypical epithelial proliferation without stromal invasion (Seidman et al. (2002) Blaustein's pathology of the female genital tract, page 791; Seidman et al. (2003) Hematol Oncol Clin North Am, 17:909). The behavior of these tumors is distinct from low-grade ovarian carcinoma, and they are considered a distinct clinical entity.

Borderline ovarian epithelial neoplasms are noninvasive neoplasms that occasionally have intraperitoneal spread. This group of neoplasms exhibits behavior that is intermediate between benign cystadenomas and invasive carcinomas. These have been referred to by different terms, including: borderline, atypical proliferative, and tumors of low malignant potential. Borderline neoplasm is currently the most widely used designation by pathologists, gynecologists, and oncologists, and has been adopted into the World Health Organization (WHO) classification.

Borderline tumors account for 14 to 15 percent of all primary ovarian neoplasms.

Borderline tumors occur in a variety of histologies, as in epithelial ovarian carcinoma. The majority of cases are serous or mucinous. In some cases, endometrioid, clear-cell, or transitional cell (Brenner) borderline tumors are found.

### Cancer therapy

The methods of the present disclosure (e.g., diagnostic and/or prognostic methods) can be followed by treating the patients using cancer therapy described herein or those known in the art, *e.g.,* standard-of-care treatments for cancer well-known to the skilled artisan, chemotherapeutic agents, hormones, antiangiogens, radiolabelled, compounds, or with surgery, cryotherapy, immunotherapy, cancer vaccine, immune cell engineering (e.g., CAR-T), and/or radiotherapy. The preceding treatment methods can be administered in conjunction with other forms of cancer therapy, either consecutively with, pre- or post-said cancer therapy. For example, immunotherapy can be administered with a therapeutically effective dose of chemotherapeutic agent, e.g., immunotherapy can be administered in conjunction with chemotherapy to enhance the activity and efficacy of the chemotherapeutic agent. The Physicians' Desk Reference (PDR) discloses dosages of chemotherapeutic agents that have been used in the treatment of various cancers. The dosing regimen and dosages of these aforementioned chemotherapeutic drugs that are therapeutically effective will depend on the particular cancer being treated, the extent of the disease and other factors familiar to the physician of skill in the art, and can be determined by the physician.

Immunotherapy is a targeted therapy that may comprise, for example, the use of cancer vaccines and/or sensitized antigen presenting cells. For example, an oncolytic virus is a virus that is able to infect and lyse cancer cells, while leaving normal cells unharmed, making them potentially useful in cancer therapy. Replication of oncolytic viruses both facilitates tumor cell destruction and also produces dose amplification at the tumor site. They may also act as vectors for anticancer genes, allowing them to be specifically delivered to the tumor site. The immunotherapy can involve passive immunity for short-term protection of a host, achieved by the administration of pre-formed antibody directed against a cancer antigen or disease antigen (*e.g.*, administration of a monoclonal antibody, optionally linked to a chemotherapeutic agent or toxin, to a tumor antigen). For example, anti-VEGF is known to be effective in treating renal cell carcinoma. Immunotherapy can also focus on using the cytotoxic lymphocyte-recognized epitopes of cancer cell lines. Alternatively, antisense polynucleotides, ribozymes, RNA interference molecules, triple helix polynucleotides and the like, can be used to selectively modulate biomolecules that are linked to the initiation, progression, and/or pathology of a tumor or cancer.

Immunotherapy also encompasses immune checkpoint modulators. Immune checkpoints are a group of molecules on the cell surface of CD4+ and/or CD8+ T cells that fine-tune immune responses by down-modulating or inhibiting an anti-tumor immune response. Immune checkpoint proteins are well-known in the art and include, without limitation, CTLA-4, PD-1, VISTA, B7-H2, B7-H3, PD-L1, B7-H4, B7-H6, 2B4, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, HHLA2, TMIDG2, KIR3DL3, and A2aR (see, for example, WO 2012/177624). Inhibition of one or more immune checkpoint inhibitors can block or otherwise neutralize inhibitory signaling to thereby upregulate an immune response in order to more efficaciously treat cancer. In some embodiments, the cancer vaccine is administered in combination with one or more inhibitors of immune checkpoints (immune checkpoint inhibition therapy), such as PD1, PD-L1, and/or CD47 inhibitors.

Adoptive cell-based immunotherapies can be combined with the therapies of the present invention. Well-known adoptive cell-based immunotherapeutic modalities, including, without limitation, irradiated autologous or allogeneic tumor cells, tumor lysates or apoptotic tumor cells, antigen-presenting cell-based immunotherapy, dendritic cell-based immunotherapy, adoptive T cell transfer, adoptive CAR T cell therapy, autologous immune enhancement therapy (AIET), cancer vaccines, and/or antigen presenting cells. Such cell-based immunotherapies can be further modified to express one or more gene products to further modulate immune responses, such as expressing cytokines like GM-CSF, and/or to express tumor-associated antigen (TAA) antigens, such as Mage-1, gp-100, and the like.

The term "chimeric antigen receptor" or "CAR" refers to engineered T cell receptors (TCR) having a desired antigen specificity. T lymphocytes recognize specific antigens through interaction of the T cell receptor (TCR) with short peptides presented by major histocompatibility complex (MHC) class I or II molecules. For initial activation and clonal expansion, naive T cells are dependent on professional antigen-presenting cells (APCs) that provide additional co-stimulatory signals. TCR activation in the absence of co-stimulation can result in unresponsiveness and clonal anergy. To bypass immunization, different approaches for the derivation of cytotoxic effector cells with grafted recognition specificity have been developed. CARs have been constructed that consist of binding domains derived from natural ligands or antibodies specific for cell-surface components of the TCR-associated CD3 complex. Upon antigen binding, such chimeric antigen receptors link to endogenous signaling pathways in the effector cell and generate activating signals similar to those initiated by the TCR complex. Since the first reports on chimeric antigen receptors, this concept has steadily been refined and the molecular design of chimeric receptors has been optimized and routinely use any number of well-known binding domains, such as scFV and another protein binding fragments described herein.

In other embodiments, immunotherapy comprises non-cell-based immunotherapies. In some embodiments, compositions comprising antigens with or without vaccine-enhancing adjuvants are used. Such compositions exist in many well-known forms, such as peptide compositions, oncolytic viruses, recombinant antigen comprising fusion proteins, and the like. In some embodiments, immunomodulatory cytokines, such as interferons, G-CSF, imiquimod, TNFalpha, and the like, as well as modulators thereof (*e.g.*, blocking antibodies or more potent or longer lasting forms) are used. In some embodiments, immunomodulatory interleukins, such as IL-2, IL-6, IL-7, IL-12, IL-17, IL-23, and the like, as well as modulators thereof (*e.g.*, blocking antibodies or more potent or longer lasting forms) are used. In some embodiments, immunomodulatory chemokines, such as CCL3, CCL26, and CXCL7, and the like, as well as modulators thereof (*e.g.*, blocking antibodies or more potent or longer lasting forms) are used. In some embodiments, immunomodulatory molecules targeting immunosuppression, such as STAT3 signaling modulators, NFkappaB signaling modulators, and immune checkpoint modulators, are used.

In still other embodiments, immunomodulatory drugs, such as immunocytostatic drugs, glucocorticoids, cytostatics, immunophilins and modulators thereof (*e.g.*, rapamycin, a calcineurin inhibitor, tacrolimus, ciclosporin (cyclosporin), pimecrolimus, abetimus, gusperimus, ridaforolimus, everolimus, temsirolimus, zotarolimus, etc.), hydrocortisone (cortisol), cortisone acetate, prednisone, prednisolone, methylprednisolone, dexamethasone, betamethasone, triamcinolone, beclometasone, fludrocortisone acetate, deoxycorticosterone acetate (doca) aldosterone, a non-glucocorticoid steroid, a pyrimidine synthesis inhibitor, leflunomide, teriflunomide, a folic acid analog, methotrexate, anti-thymocyte globulin, antilymphocyte globulin, thalidomide, lenalidomide, pentoxifylline, bupropion, curcumin, catechin, an opioid, an IMPDH inhibitor, mycophenolic acid, myriocin, fingolimod, an NF-xB inhibitor, raloxifene, drotrecogin alfa, denosumab, an NF-xB signaling cascade inhibitor, disulfiram, olmesartan, dithiocarbamate, a proteasome inhibitor, bortezomib, MG132, Prol, NPI-0052, curcumin, genistein, resveratrol, parthenolide, thalidomide, lenalidomide, flavopiridol, non-steroidal anti-inflammatory drugs (NSAIDs), arsenic trioxide, dehydroxymethylepoxyquinomycin (DHMEQ), I3C(indole-3-carbinol)/DIM(di-indolmethane) (13C/DIM), Bay 11-7082, luteolin, cell permeable peptide SN-50, IKBa.-super repressor overexpression, NFKB decoy oligodeoxynucleotide (ODN), or a derivative or analog of any thereo, are used. In yet other embodiments, immunomodulatory antibodies or protein are used. For example, antibodies that bind to CD40, Toll-like receptor (TLR), OX40, GITR, CD27, or to 4-1BB, T-cell bispecific antibodies, an anti-IL-2 receptor antibody, an anti-CD3 antibody, OKT3 (muromonab), otelixizumab, teplizumab, visilizumab, an anti-CD4 antibody, clenoliximab, keliximab, zanolimumab, an anti-CD11 a antibody, efalizumab, an anti-CD18 antibody, erlizumab, rovelizumab, an anti-CD20 antibody, afutuzumab, ocrelizumab, ofatumumab, pascolizumab, rituximab, an anti-CD23 antibody, lumiliximab, an anti-CD40 antibody, teneliximab, toralizumab, an anti-CD40L antibody, ruplizumab, an anti-CD62L antibody, aselizumab, an anti-CD80 antibody, galiximab, an anti-CD147 antibody, gavilimomab, a B-Lymphocyte stimulator (BLyS) inhibiting antibody, belimumab, an CTLA4-Ig fusion protein, abatacept, belatacept, an anti-CTLA4 antibody, ipilimumab, tremelimumab, an anti-eotaxin 1 antibody, bertilimumab, an anti-a4-integrin antibody, natalizumab, an anti-IL-6R antibody, tocilizumab, an anti-LFA-1 antibody, odulimomab, an anti-CD25 antibody, basiliximab, daclizumab, inolimomab, an anti-CD5 antibody, zolimomab, an anti-CD2 antibody, siplizumab, nerelimomab, faralimomab, atlizumab, atorolimumab, cedelizumab, dorlimomab aritox, dorlixizumab, fontolizumab, gantenerumab, gomiliximab, lebrilizumab, maslimomab, morolimumab, pexelizumab, reslizumab, rovelizumab, talizumab, telimomab aritox, vapaliximab, vepalimomab, aflibercept, alefacept, rilonacept, an IL-1 receptor antagonist, anakinra, an anti-IL-5 antibody, mepolizumab, an IgE inhibitor, omalizumab, talizumab, an IL12 inhibitor, an IL23 inhibitor, ustekinumab, and the like.

Nutritional supplements that enhance immune responses, such as vitamin A, vitamin E, vitamin C, and the like, are well-known in the art (see, for example, U.S. Pat. Nos. 4,981,844 and 5,230,902 and PCT Publ. No. WO 2004/004483) can be used in the methods described herein.

Similarly, various agents or a combination thereof can be used to treat a cancer. For example, chemotherapy, radiation, epigenetic modifiers (*e.g.*, histone deacetylase (HDAC) modifiers, methylation modifiers, phosphorylation modifiers, and the like), targeted therapy, and the like are well-known in the art.

In some embodiments, chemotherapy is used. Chemotherapy includes the administration of a chemotherapeutic agent. Such a chemotherapeutic agent may be, but is not limited to, those selected from among the following groups of compounds: platinum compounds, cytotoxic antibiotics, antimetabolites, anti-mitotic agents, alkylating agents, arsenic compounds, DNA topoisomerase inhibitors, taxanes, nucleoside analogues, plant alkaloids, and toxins; and synthetic derivatives thereof. Exemplary compounds include, but are not limited to, alkylating agents: cisplatin, treosulfan, and trofosfamide; plant alkaloids: vinblastine, paclitaxel, docetaxol; DNA topoisomerase inhibitors: teniposide, crisnatol, and mitomycin; anti-folates: methotrexate, mycophenolic acid, and hydroxyurea; pyrimidine analogs: 5-fluorouracil, doxifluridine, and cytosine arabinoside; purine analogs: mercaptopurine and thioguanine; DNA antimetabolites: 2'-deoxy-5-fluorouridine, aphidicolin glycinate, and pyrazoloimidazole; and antimitotic agents: halichondrin, colchicine, and rhizoxin. Compositions comprising one or more chemotherapeutic agents (*e.g.*, FLAG, CHOP) may also be used. FLAG comprises fludarabine, cytosine arabinoside (Ara-C) and G-CSF. CHOP comprises cyclophosphamide, vincristine, doxorubicin, and prednisone. In another embodiments, PARP (*e.g.*, PARP-1 and/or PARP-2) inhibitors are used and such inhibitors are well-known in the art (*e.g.*, Olaparib, ABT-888, BSI-201, BGP-15 (N-Gene Research Laboratories, Inc.); INO-1001 (Inotek Pharmaceuticals Inc.); PJ34 (Soriano *et al.*, 2001; Pacher *et al.*, 2002b); 3-aminobenzamide (Trevigen); 4-amino-1,8-naphthalimide; (Trevigen); 6(5H)-phenanthridinone (Trevigen); benzamide (U.S. Pat. Re. 36,397); and NU1025 (Bowman *et al.*). The mechanism of action is generally related to the ability of PARP inhibitors to bind PARP and decrease its activity. PARP catalyzes the conversion of .beta.-nicotinamide adenine dinucleotide (NAD+) into nicotinamide and poly-ADP-ribose (PAR). Both poly (ADP-ribose) and PARP have been linked to regulation of transcription, cell proliferation, genomic stability, and carcinogenesis (Bouchard V. J. et.al. Experimental Hematology, Volume 31, Number 6, June 2003, pp. 446-454(9); Herceg Z.; Wang Z.-Q. Mutation Research/Fundamental and Molecular Mechanisms of Mutagenesis, Volume 477, Number 1, 2 Jun. 2001, pp. 97-110(14)). Poly(ADP-ribose) polymerase 1 (PARP1) is a key molecule in the repair of DNA single-strand breaks (SSBs) (de Murcia J. et al. 1997. Proc Natl Acad Sci USA 94:7303-7307; Schreiber V, Dantzer F, Ame J C, de Murcia G (2006) Nat Rev Mol Cell Biol 7:517-528; Wang Z Q, et al. (1997) Genes Dev 11:2347-2358). Knockout of SSB repair by inhibition of PARP1 function induces DNA double-strand breaks (DSBs) that can trigger synthetic lethality in cancer cells with defective homology-directed DSB repair (Bryant H E, et al. (2005) Nature 434:913-917; Farmer H, et al. (2005) Nature 434:917-921). The foregoing examples of chemotherapeutic agents are illustrative, and are not intended to be limiting.

In other embodiments, radiation therapy is used. The radiation used in radiation therapy can be ionizing radiation. Radiation therapy can also be gamma rays, X-rays, or proton beams. Examples of radiation therapy include, but are not limited to, external-beam radiation therapy, interstitial implantation of radioisotopes (I-125, palladium, iridium), radioisotopes such as strontium-89, thoracic radiation therapy, intraperitoneal P-32 radiation therapy, and/or total abdominal and pelvic radiation therapy. For a general overview of radiation therapy, see Hellman, Chapter 16: Principles of Cancer Management: Radiation Therapy, 6th edition, 2001, DeVita et al., eds., J. B. Lippencott Company, Philadelphia. The radiation therapy can be administered as external beam radiation or teletherapy wherein the radiation is directed from a remote source. The radiation treatment can also be administered as internal therapy or brachytherapy wherein a radioactive source is placed inside the body close to cancer cells or a tumor mass. Also encompassed is the use of photodynamic therapy comprising the administration of photosensitizers, such as hematoporphyrin and its derivatives, Vertoporfin (BPD-MA), phthalocyanine, photosensitizer Pc4, demethoxy-hypocrellin A; and 2BA-2-DMHA.

In other embodiments, hormone therapy is used. Hormonal therapeutic treatments can comprise, for example, hormonal agonists, hormonal antagonists (*e.g.*, flutamide, bicalutamide, tamoxifen, raloxifene, leuprolide acetate (LUPRON), LH-RH antagonists), inhibitors of hormone biosynthesis and processing, and steroids (*e.g.*, dexamethasone, retinoids, deltoids, betamethasone, cortisol, cortisone, prednisone, dehydrotestosterone, glucocorticoids, mineralocorticoids, estrogen, testosterone, progestins), vitamin A derivatives (*e.g.*, all-trans retinoic acid (ATRA)); vitamin D3 analogs; antigestagens (*e.g.*, mifepristone, onapristone), or antiandrogens (*e.g.*, cyproterone acetate).

In other embodiments, photodynamic therapy (also called PDT, photoradiation therapy, phototherapy, or photochemotherapy) is used for the treatment of some types of cancer. It is based on the discovery that certain chemicals known as photosensitizing agents can kill one-celled organisms when the organisms are exposed to a particular type of light.

In yet other embodiments, laser therapy is used to harness high-intensity light to destroy cancer cells. This technique is often used to relieve symptoms of cancer such as bleeding or obstruction, especially when the cancer cannot be cured by other treatments. It may also be used to treat cancer by shrinking or destroying tumors.

### Clincal Efficacy / Response to a Therapy

Clinical efficacy can be measured by any method known in the art. For example, the response to a therapy relates to any response of the cancer, *e.g.,* a tumor, to the therapy, preferably to a change in tumor mass and/or volume after initiation of neoadjuvant or adjuvant chemotherapy. Tumor response may be assessed in a neoadjuvant or adjuvant situation where the size of a tumor after systemic intervention can be compared to the initial size and dimensions as measured by CT, PET, mammogram, ultrasound or palpation and the cellularity of a tumor can be estimated histologically and compared to the cellularity of a tumor biopsy taken before initiation of treatment. Response may also be assessed by caliper measurement or pathological examination of the tumor after biopsy or surgical resection. Response may be recorded in a quantitative fashion like percentage change in tumor volume or cellularity or using a semi-quantitative scoring system such as residual cancer burden (Symmans et al., J. Clin. Oncol. (2007) 25:4414-4422) or Miller-Payne score (Ogston et al., (2003) Breast (Edinburgh, Scotland) 12:320-327) in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of tumor response may be performed early after the onset of neoadjuvant or adjuvant therapy, *e.g.*, after a few hours, days, weeks or preferably after a few months. A typical endpoint for response assessment is upon termination of neoadjuvant chemotherapy or upon surgical removal of residual tumor cells and/or the tumor bed.

In some embodiments, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments, the CBR for a particular anti-immune checkpoint therapeutic regimen is at least 25%, 30%, 35%, 40%, 45%, 50%, 55%, 60%, 65%, 70%, 75%, 80%, 85%, or more.

Additional criteria for evaluating the response to a cancer therapy are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.*, time of diagnosis or start of treatment) and end point (*e.g.*, death, recurrence or metastasis). In addition, criteria for efficacy of treatment can be expanded to include probability of survival, probability of metastasis within a given time period, and probability of tumor recurrence.

For example, in order to determine appropriate threshold values, a particular anti-cancer therapeutic regimen can be administered to a population of subjects and the outcome can be correlated to biomarker measurements that were determined prior to administration of any cancer therapy. The outcome measurement may be pathologic response to therapy given in the neoadjuvant setting. Alternatively, outcome measures, such as overall survival and disease-free survival can be monitored over a period of time for subjects following the cancer therapy for whom biomarker measurement values are known. In certain embodiments, the same doses of anti-cancer agents are administered to each subject. In related embodiments, the doses administered are standard doses known in the art for anti-cancer agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker measurement threshold values that correlate to outcome of a cancer therapy can be determined using methods such as those described in the Examples section.

### EXAMPLES

### Example 1: Materials and methods

### Patients

Patients samples (TMAs and liquid biopsies) were obtained from the LDI/JHG biobank under ethical approval. Retrospective study of clinical history was used to asses the correlation between TMGs and CA-125 levels, cancer disease stages and survival, and menopausal stage. A total of 176 patients were studied (n = 9 normal; n = 16 borderline; n = 151 ovarian).

### Extraction and isolation of gangliosides from tissues

Total lipids from tissues (e.g., tumors) (500 mg) that have been homogenized are extracted with 9 ml chloroform-methanol (2:1; v/v) and then with 7.6 ml chloroform-methanol-water (1:2:0.8; v/v/v). The extraction is dried under a gentle stream of nitrogen, re-dissolved with 7.5 ml chloroform-methanol-water (30:60:8; v/v/v), and applied to a DEAE-Sephadex A-25 column (6 × 45 mm, acetate form) (Amersham Biosciences AB, Sweden). After washing neutral lipids from the column with 15 ml chloroform-methanol-water (30:60:8; v/v/v), the acidic glycolipid mixtures containing gangliosides are eluted with 7.5 ml chloroform-methanol-0.8 M sodium acetate (30:60:8; v/v/v).

### Extraction and isolation of gangliosides from liquid biopsy

Monophasic lipid extraction of polar gangliosides with a solvent volumes ratio of 0.85 : 2 : 1 : 0.75 MeOH : EtOH : CHCl3 : H2O. The method has been adapted from Svennerholm and Fredman (1980) Biochim Biophys Acta 617:97-109; and Lydic et al. (2014) J Lipid Res 55:1797-809, both of which are incorporated by reference in their entirety.

### Liquid chromatography/electrospray ionization-MS

Liquid chromatography (LC) separations are achieved by using an octadecyl silane (ODS) reverse-phase LC (RPLC) column (150 × 0.3 mm inner diameter) (LC PACKINGS; Amsterdam, The Netherlands) at 45°C. The mobile phase is prepared by methanol (A), isopropanol (B), and water (containing 200 mM ammonium formate) (C), and the composition is produced by mixing these solvents. The gradient consist of holding solvent (A/B/C 55:25:20) (D) for 5 min, then linearly converting solvent D to solvent (A/B 60:40) (E) for 50 min, and holding solvent E for 35 min. The mobile phase is pumped at a flow rate of 7.5 µl/min for the gradient elution. Typically, 3 µl of sample solution is applied.

### Electrospray ionization-tandem mass spectrometry analysis

The electrospray ionization (ESI)-MS analyses are performed by using a 4000Q TRAP quadrupole-linear ion trap hybrid MS (Applied Biosystems, Foster City, CA) with an UltiMate 3000 nano/cap/micro LC system (Dionex Corporation, Sunnyvale, CA) combined with an HTS PAL autosampler (CTC Analytics AG, Zwingen, Switzerland).

The scan range of the instrument is set from *m*/*z* 200 to 2,300 at a scan speed of 1,000 amu/s. The trap fill time is 10 ms in the positive-ion mode and 20 ms in the negative-ion mode. Other MS scan conditions are operated with the ion spray voltage of 5,500 V, declustering potential (DP) of 80 V, and collision-induced dissociation (CID) of 10 V in the positive-ion mode, and with the ion spray voltage of -4,500 V, DP of -120 V, and CID of -5 V in the negative-ion mode. Tandem mass spectrometry (MS/MS) analyses of ceramide molecular species derived from in-source decay of gangliosides are performed under each CID condition: d16:1-18:0/d18:1-16:0 (60 V), d18:1-18:0 and d20:1-18:0 (85 V), and d20:1-20:0/d18:1-22:0 (75 V).

### Multiple reaction monitoring (MRM) analysis

MRM analyses are performed with the same instrument system used for the LC/ESI-MS analysis. DP is set at -120 V, and CID conditions for each ganglioside are optimized as follows: GM1, GM2, and GM3 (-95 V), GD1, GD2, and GD3 (-55 V), GT1, GT2, GT3, and GQ1 (-60 V). The precursor and product ion pairs for the MRM analysis are selected by these MS/MS spectra. The following MRM *m*/*z* transitions are monitored: parent ions to sialic acid ions (SA⁻) or sialic acid-containing sugar chains for gangliosides. For the analysis of regioisomeric gangliosides, specific fragments derived from terminal regions of the sugar chain in these isomers are selected and operated under the same conditions of the MRM analysis for each ganglioside.

### Example 2: LC-MS assay in liquid biopsies - level of gangliosides

Using nLC-ESI-MS/MS (LC-MS), liquid biopsies from 6 cancer patients (2 Melanoma, 2 Renal cancer, 2 Small Cell Lung Cancer (SCLC_ newly diagnosed-treatment naïve) and 3 non-cancer controls (2 donors studied individually, and 1 pooled plasma from 30 donors) were analyzed (Tables 5 and 6). The LC-MS method was developed to study all tumor gangliosides at once (e.g. the Cancer Gangliosome Matrix, comprising 20 tumor gangliosides within at least 5,000 analytes).

Cancer patients showed elevated tumor gangliosides, which were specific to each cancer. Assesed by LC-MS, and compared to non-cancer samples, melanoma samples showed a significant increase in the level of GM2, GD3 and GD1b; renal cancer samples showed a significant increase in the level of GD3 and GD2; and lung cancer samples showed a significant increase in the level of GD3 and GM2. In the studies of ovarian cancer samples done specifically to evaluate GD3, 12/13 ovarian cancer samples demonstrated a significant increase in GD3 compared to 2 non-cancer control samples. Control non-cancer samples showed the level that was very low or below the threshold of detection (Table 1). Internal standards included Cholesterol (not shown) and Phosphatidylcholine (not shown) which were present at high levels in all samples. Phosphatidylserine (PS), a non-specific positive marker of general stress (cancer, inflammation, diabetes, infection, sepsis, apoptosis), was elevated in all cancer samples over control non-cancer samples.

**Table 1: LC-MS-MS detection of gangliosides as diagnostics of cancer.**

| **Tumor Ganglioside** | **Melanoma** | **Renal Cancer** | **Lung Cancer** | **Non-cancer** | | **OvarianCancer** | **Non-cancer** |
|---|---|---|---|---|---|---|---|
| GM2 | 1,350 | 60 | 140 | 14 | | | |
| GD3 | 430 | 200 | 100 | 10 | | 213 | 33 |
| GD2 | 45 | 155 | 20 | 15 | | | |
| GD1b | 80 | 0 | 0 | 0 | | | |
| PS | 60,000 | 26,000 | 18,000 | 4,000 | | | |

Data is shown with relative quantification. The TMGs in serum of cancer patients have discrete lengths of their lipid chains, different from cancer to cancer. Lengths can vary from 16 to 44 Carbons, so this feature was analyzed in detail.

Left Panel (melanoma, renal cancer, lung cancer, and non-cancer) is 1 patient per case, with relative quantification. The limit of Quantifiable Detection (LOQD) was ~1-20 units. Right Panel (ovarian cancer and non-cancer) is absolute quantification (pmol/ml). Glycolipids, measured in serum that was taken at the time of diagnosis, compared the average of thirteen ovarian cancer samples (4 early stage and 9 late stage) to the average of 2 non-cancer samples.

Fig. 2 further shows GD3 analytes in early and late stage ovarian cancer versus non-cancer (* p < 0.05 and ** p< 0.01).

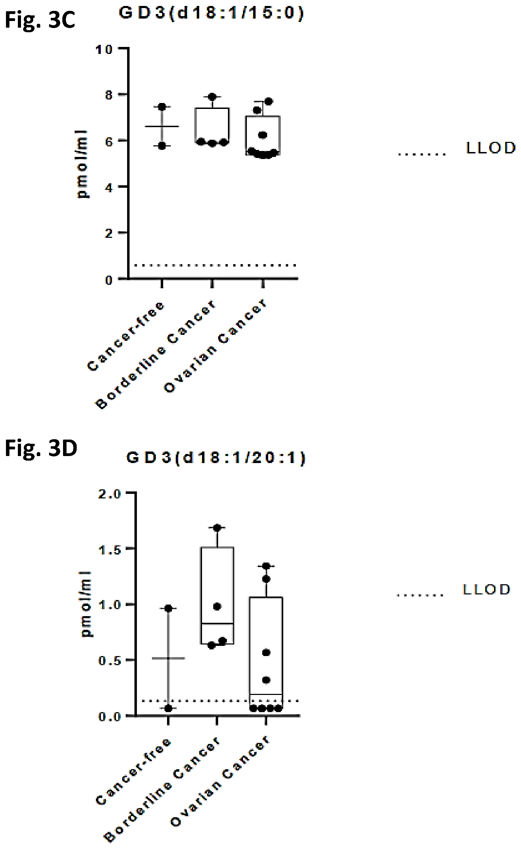

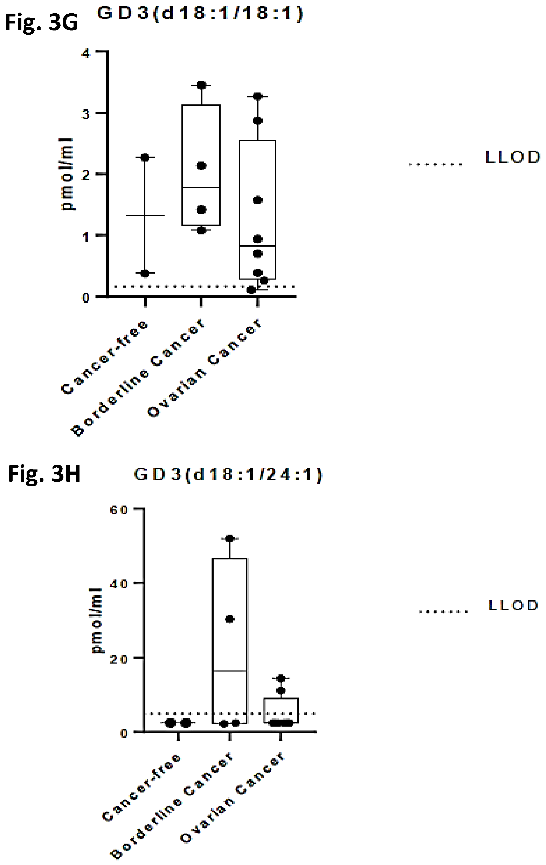

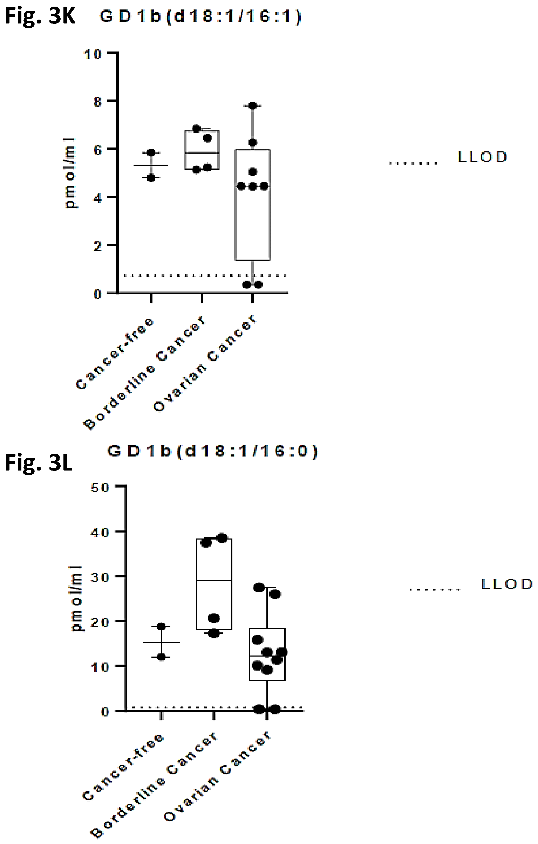

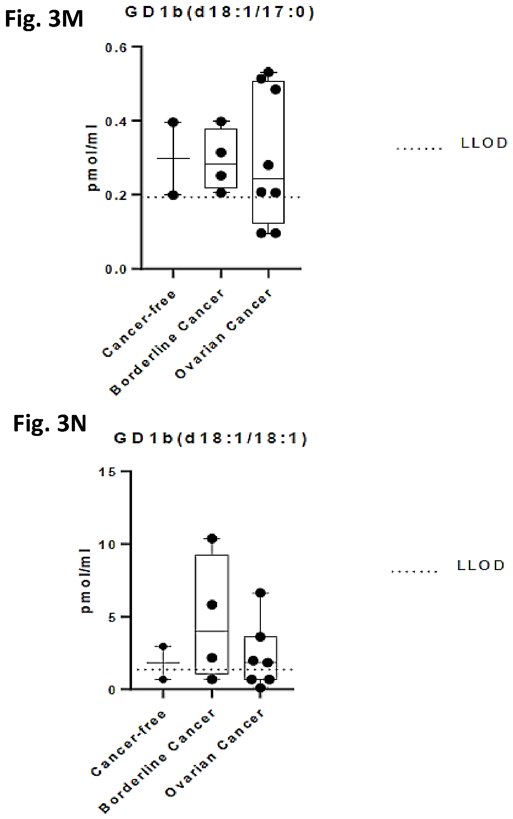

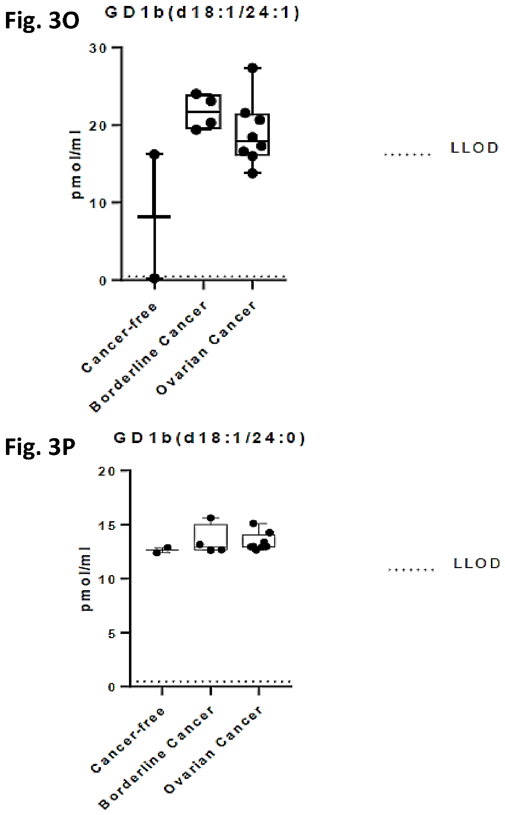

In both data sets (Table 1 and Fig. 2), the tumor gangliosides in serum differ quantitatively in terms of total quantification depending on whether non-cancer or cancer samples are evaluated (with TMGs being elevated in cancer). Fig. 3 also shows that TMGs also differ in terms of their lipid chain lengths which are qualitatively and quantitatively different depending on whether non-cancer or cancer samples are evaluated.

### Example 3: LC-MS assay in liquid biopsies - lipid tail length

In addition to demonstrating by LC-MS that cancer patients have elevated levels of tumor gangliosides, provided herein is a surprising and unexpected finding that the tumor gangliosides have lipid tail heterogeneity patterns that are specific to each cancer.

In melanoma, GM2 with the short chain forms (lipid tails) predominated and were increased 10-fold above normal, whereas the long chain forms increased from undetectable in normal to 90 units in cancer. For GD3, the short chain forms increased 40-fold above normal. For GD1 short chain forms increased from undetectable in normal to 70 units. Renal cancer had significant increases in GD3 and GD2 (particularly in short lipid chain forms). Lung cancer had significant increases or shifts in GD3 (particularly in long lipid chain forms) and a shift in GM2 to short lipid chain forms (Table 2). All control samples had signatures very low or below threshold, but normal gangliosides such as GM1 were detected.

As demonstrated herein, using LC-MS, the present disclosure detected and quantified the lipid variable length of gangliosides, and established the association of the lipid tail length with the cancer diagnosis. In addition, as further demonstrated herein, the LC-MS method detected other ganglioside tumor markers such as GM2 and GD1, which currently cannot be quantified in ELISA. Detection of a specific signature for many tumor gangliosides (e.g. the Cancer Gangliosome Matrix) is of value for expanded applications and detection/diagnosis/prognosis of tumors.

**Table 2: LC-MS-MS detection of gangliosides as diagnostic of human cancer.**

| **Analyte** | **Melanoma** | | **Renal Cancer** | | **Lung Cancer** | | **Non-cancer controls** | |
|---|---|---|---|---|---|---|---|---|
| | **Short lipid** | **Long lipid** | **Short lipid** | **Long lipid** | **Short lipid** | **Long lipid** | **Short lipid** | **Long lipid** |
| GM2 | **1260** | **90** | 0 | **60** | 20 | **120** | 140 | 0 |
| GD3 | **380** | 50 | **110** | **90** | 30 | **70** | 10 | 10 |
| GD2 | 30 | 15 | **155** | **0** | 15 | 5 | 15 | 60 |
| GD1 | **70** | 10 | 0 | 0 | 0 | 0 | 0 | 0 |

Glycolipids and lipids of serum were studied at time of diagnosis. Only relevant data compared to normal controls are shown, in relative units. For simplicity, the lipid tails are segregated as short (14-24 carbons) or long (26-38 carbons). Examples shown include a melanoma with extensive metastatic disease; a kidney cancer, and a non-small cell lung cancer. Patients were compared to the average of non-cancer donors. Example of data from ~5,000 analytes (significant changes vs. normal in bold). Other gangliosides remain unchanged or undetectable. The currently estimated Limit of Quantifiable Detection (LOQD) is ~40 units, or 0.010-0.24 pmol/mL.

### Example 4: LC-MS assay in liquid biopsies identified GD3 with an acyl chain of 16-24 carbons in length as an important biomarker for ovarian cancers

To address the need to quantify ganglioside levels and -moreover- to identify the molecular identities of potential biomarkers, a reversed-phase liquid chromatography- mass spectrometry method was applied to gangliosides extracted form donors with cancer or normal healthy individuals. Gangliosides from the serum samples were purified using standard methods. In the present case we used addition of 5-fold volume of cold Methanol, centrifugation to precipitate proteins and undesired molecules, and collection of the monophasic supernatant containing gangliosides.

The total levels of GD3 were assessed, and the lipid species of GD3 was evaluated. The data indicate that (a) the total concentration (in nM) of GD3 was increased in cancer, and (b) that the increased GD3 are species with short acyl-lipid chains, ranging from C16-C24 in length. The short acyl chain is as defined in caption to **Table 2** (see above). These data indicate that a lipid-species can be identified as it is increased in cancer, and measurement methods may be used to identify a cancer. In the present example, GD3 species with short acyl chains have been identified as being increased in ovarian cancer.

**Table 3: LC-MS/MS detection of gangliosides as diagnostic of human cancer.**

| **serum source** | **Total GD3 (nM)** | **GD3 C16-C24 acyl chain fold-increase** |
|---|---|---|
| **OVCA cancer** | 1025.40 | 2.4 |
| **Benign tumors** | 448.05 | 0.7 |
| **Normal** | 632.45 | 1 |

Total GD3 in serum is increased in ovarian cancer (OVCA) compared to serum collected form patients with benign gynecological tumors or from normal healthy individuals (each n=5, averaged). The acyl chain of GD3 increased in OVCA has a range of C16-C24 carbon-length (defined and categorized herein as "short" lipid) and is shown as a 2.4-fold increase in cancer (standardized to Normal).

### Example 5: LC-ESI-MS/MS assay in liquid biopsies identified certain lipoforms, e.g., GD3(d18:1/23:0), as an important biomarker for ovarian cancers

To address the need to quantify ganglioside levels and identify the molecular identities of potential biomarkers, a reversed-phase nanobore liquid chromatography-electrospray ionization tandem mass spectrometry (nLC-ESI-MS/MS) method for disialogangliosides GD1b, GD2 and GD3. Levels of all GD3, GD1 and GD1b species with one exception were comparable between all groups.

Remarkably, GD3(d18:1/23:0) level was below the lower limit of detection in healthy controls but, robustly detected at high abundance in serum of both ovarian cancer patients and borderline tumor patients.

**Table 4: LC-ESI-MS/MS detection and quantification of disialogangliosides (GDs). Serum was collected at time of diagnosis. Quantitation of GD1b, GD2 and GD3 molecular lipid species was performed using calibration curves and regression equations obtained from measuring each of GD1b, GD2 and GD3 lipid species in standard solutions. Molecular identities were confirmed by LC-selected reaction monitoring (SRM)-information dependent acquisition (IDA)-enhanced product ion (EPI) experiments in which**

| **Analyte** | **LLOD (pmol/ml)** | **LLOQ (pmol/ml)** | **ULOQ (pmol/ml)** | **No Cancer (Control)** | **Ovarian Cancer** | **Borderline tumor** | ***p*-value (ANOV A with Welch correct ion)** |
|---|---|---|---|---|---|---|---|
| GD3(d18:1/ 14:0) | 0.574 | 0.861 | 587.675 | 9.115 | 8.775 | 9.186 | 0.8809 |
| GD3(d18:1/ 15:0) | 0.577 | 0.865 | 590.590 | 6.615 | 6.051 | 6.409 | 0.7978 |
| **GD3(d18:1/ 16:0)** | 5.186 | 7.778 | 5310.054 | 14.906 | 23.175 | 36.582 | 0.5085 |
| GD3(d18:1/ 18:1) | 0.164 | 0.246 | 168.062 | 1.325 | 1.266 | 2.021 | 0.6407 |
| GD3(d18:1/ 20:1) | 0.134 | 0.201 | 137.068 | 0.963 | 0.864 | 0.992 | 0.4211 |
| **GD3(d18:1/ 23:0)** | 42.756 | 64.134 | 43782.460 | <LLOD | 127.816 | 128.860 | 0.0005 * |
| **GD3(d18:1/ 24:1)** | 4.968 | 7.451 | 5086.827 | <LLOD | 12.811 | 28.223 | 0.2722 |
| **GD2(d18:1/ 16:0)** | 11.783 | 17.674 | 12065.640 | 26.726 | 36.792 | 48.351 | 0.2789 |
| GD1b(d18:1 /13:1) | 0.259 | 0.388 | 264.710 | 1.387 | 1.047 | 2.966 | 0.0765 |
| GD1b(d18:1 /16:1) | 0.729 | 1.094 | 746.501 | 5.321 | 5.411 | 5.915 | 0.3385 |
| **GD1b(d18: 1/16:0)** | 0.729 | 1.094 | 746.501 | 15.365 | 15.747 | 28.475 | 0.1803 |
| GD1b(d18:1 /17:0) | 0.193 | 0.289 | 197.339 | 0.297 | 0.371 | 0.292 | 0.9932 |
| GD1b(d18:1 /18:1) | 1.353 | 2.029 | 1385.210 | 2.958 | 2.832 | 4.766 | 0.5846 |
| **GD1b(d18: 1/24:1)** | 0.453 | 0.680 | 464.213 | 16.218 | 18.983 | 21.730 | 0.3274 |
| GD1b(d18:1 /24:0) | 0.453 | 0.680 | 464.213 | 12.626 | 13.385 | 13.510 | 0.2183 |
| **Total GD3** | | | | 32.924 | 181.036 | 213.491 | 0.0021 * |
| **Total GD2** | | | | 26.726 | 36.792 | 48.351 | 0.2823 |
| **Total GD1b** | | | | 54.173 | 57.775 | 77.655 | 0.2834 |

the SRM was used as a survey scan to identify target analytes and an IDA-EPI scan was acquired in the linear ion trap allowing for complete structure characterization of each ganglioside species. For statistical purposes, where control values for GD3(d18:1/23:0) fell below LLOD values, they were assigned a value 2-fold lower than the LLOD.

### Example 6: LC/MS liquid biopsy test for pan-cancer screening based on quantification of tumor gangliosides

The main challenge in cancer research is the early detection of tumors when treatment therapies are more likely to be successful. For certain cancers, it may take decades for a tumor to progress to late-stage disease, while in others, it takes just months.1,2 Early detection offers a great opportunity for intervention before cancers progress to late-stage disease or metastasize to distant sites or acquire additional mutations or heterogeneity, which make the cancer harder or impossible to treat. However, currently available routine cancer screening tests detect only five cancer types: breast, lung, colorectal, cervical and prostate, that too with limited sensitivity and specificity. This short list highlights the lack of effective early screening tests for all other types of cancer.3

Recent technologies focused on genomics (using next-generation sequencing for circulating tumour DNA) and proteomics, enabled liquid biopsy blood tests that detect signals from multiple cancers. This is referred to as multi-cancer early detection (MCED), some with promising preliminary screening outcomes such as, CancerSEEK, PanSeer and Galleri.4-7

However, the diagnostic performance of these tests is not optimal, especially at an early-stage cancer. As an example, the Galleri's overall sensitivity across 12 cancer types and stages was 51.5% (39% stage I to 92% stage IV).7 These tests have not yet been shown to improve clinical outcomes.8 Modeling performance of MCED tests in a representative population suggests that they may substantially reduce overall cancer mortality by stage shifting diagnosis if added to usual care.9 The key barrier for these emerging liquid biopsy-based diagnostic MCED technologies is that they must have very high specificity, to minimize overtreatment with sufficiently high sensitivity to improve outcomes.8

Presented herein are liquid biopsy assays based on liquid chromatography-mass spectrometry (LC-MS) technology. The assay involves a novel liquid biopsy method of quantification of a never before exploited family of biomarkers called tumor marker glycolipids (TMG) from blood sample. TMGs have a persistent and homogeneous expression almost exclusively in cancer, expression does not downregulate, and have etiological roles in cancer that make them ideal targets to be exploited as biomarkers. While genomics and proteomics have been used as tools in precision medicine; we foresee glycomics of TMGs as the next frontier used in diagnostics and screening.

### Tumor Marker Glycolipids (TMG)

All cells are covered with a dense coat of glycans which are chains of carbohydrates of variable length and structure, which can be bound to proteins or to lipids.10-12 Glycolipids are lipids with a carbohydrate chain covalently attached. Glycolipids can be a component of the cell membrane, where the lipid is in the membrane and the carbohydrate chain is exposed in the outside. Glycolipids are found on the surface of all eukaryotic cell membranes.10-13 An essential role of glycolipids is to maintain cell membrane stability and facilitate cellular recognition, cell-to-cell communication, tissue formation,11,14,15 and immune responses.15 A subset of glycolipids are the Gangliosides, which have a carbohydrate head tree that contains at least one sialic acid, the carbohydrate is attached to a sphingosine ceramide that contains 2 lipid tails embedded on the membrane.

Gangliosides are a family of >40 different sialic acid-containing glycosphingolipids. Each glycan head tree is structurally unique and defines each ganglioside by name. Some gangliosides are ubiquitous and are present in normal cells, whereas other gangliosides are tumor-markers, which are low/absent in normal cells and expressed at high levels in embryonic tissue and in cancer.16-22

Changes in the expression of certain species of gangliosides have been described to occur during cell proliferation, differentiation, and oncogenesis.17 Aberrant and elevated expression of gangliosides has been also observed in different types of cancer cells. A subset of gangliosides, referred to as TMGs, comprises a family of about 20 different gangliosides that are present preferentially or almost exclusively and at high density on the cell surface of certain cancers (e.g. the Cancer Gangliosome Matrix (CGM)).11

As the term "tumor marker" suggests, TMG expression is tightly associated with malignancy. Expression of tumor gangliosides provides a survival advantage to the tumor, as some TMGs are known to afford tumors with immune evasion or immunosuppression, growth-factor independent growth, and better blood supply, all of which promote tumor growth, metastasis, and survival.17 Moreover, gangliosides are actively released from the membrane of tumor cells, systemically impairing anti-tumor immunity.17

**Table 5: Tumor Marker Ganglioside Targets. Adapted from Bartish et al.11 Selected cancers where there is evidence for TMG expression in >50% of all patients in the indicated malignancy, exemplifying the prevalence of TMGs. The cells with no entry reflect ≤50% prevalence, or that we omitted literature that we deem unreliable because very few biopsies were phenotyped. The list ranges from ~95% (Neuroblastoma), to ~80% (Melanoma), to ~50% (Head and Neck) of patients. When expressed in a patient the TMGs are present homogeneously in tumor nodules and cells.**

| **Malignancy** | **GD2** | **GD3** | **Fucosyl -GM1** | **GM2** | **GM3** | **PolySia^{2 3}** | **Sialyl LewisX** |
|---|---|---|---|---|---|---|---|
| **Neuroblastoma** | ²⁴⁻²⁶ | ²⁶ | ²⁷ | ^{28,29} | ³⁰ | ^{31,32} | |
| **Melanoma** | ²⁶ | ^{26,33} | ³⁴ | ³⁵ | ³⁶ | | |
| **Glioma** | ³⁷ | ³⁸⁻⁴⁰ | | ^{23,41} | ⁴²⁻⁴⁴ | ⁴⁵ | |
| **Non-small cell lung cancer (NSCLC)** | | | ⁴⁶ | ⁴⁷ | ^{48,49} | ^{50,51} | ^{52,53} |
| **Small cell lung cancer (SCLC)** | ⁵⁴ | ⁵⁴ | ^{46,54,55} | ⁵⁴ | | | |
| **Breast carcinoma** | ⁵⁶ | ⁵⁶⁻⁵⁸ | | ⁵⁶ | ⁵⁶ | ⁵⁹ | ^{52,53} |
| **Renal cell cancer** | | | ⁶⁰ | ⁶¹ | ⁶² | | ⁶³ |
| **Ovarian cancer** | ⁶⁴ | ⁶⁵ | ⁶⁴ | ⁶⁴ | ⁶⁶ | | |
| **Soft tissue sarcomas** | ⁶⁷ | ⁶⁷ | | | | | |
| **Osteosarcoma** | ⁶⁸ | ^{69,70} | | | | | |
| **Ewing's sarcoma** | ^{71,72} | ⁷⁰ | | | ⁷³ | | |
| **Desmoplastic Round Cell** | ⁷⁴ | ⁷⁰ | | | | | |
| **Rhabdomyosarc.** | ⁷⁰ | ⁷⁰ | | | | | |
| **Retinoblastoma** | ⁷⁵ | | | | ⁷⁶ | | |
| **Willms tumor** | | | | | ^{62,77} | ⁷⁸ | |
| **Medullary thyroid cancer** | | | | | | ⁷⁹ | |
| **Prostate Cancer** | | | | ⁸⁰ | | | |
| **Gastric cancer** | | | | ⁸⁰ | | ⁸⁰ | ⁵² |
| **Endometrial** | | | | ⁸⁰ | | ^{23,80} | |
| **Pancreatic** | | | | ⁸⁰ | | ⁸⁰ | ⁵² |
| **Colon Cancer** | | | | ⁸⁰ | | | ^{52,53} |
| **Esophageal** | | | | | | | ⁵² |
| **Head and neck** | | | | | | | ^{52,53} |

Furthermore, since TMGs can be shed from the tumor membrane 81-89, the TMGs are present into extracellular fluids and can be detected in serum as well. Surprisingly, however, while TMGs appear to be ideal and high value etiological biomarkers in tissue or in serum, TMGs have been underexploited for diagnosis of any cancer.

### Liquid chromatography-mass spectrometry (LC-MS)

The LC-MS method allows for the analyses of multiple ganglioside analytes, therefore all tumor marker gangliosides can be evaluated, including GD2, GD3, GD1b, GT1b, fucosyl-GM1, GloboH, polysialic acid, GM2, GM3, sialyl-LewisX, sialyl-LewisY, sialyl-LewisA, sialyl-LewisB, and LewisY. Moreover, LC-MS allows for examination of the physical properties of the tumor gangliosides, such as the ganglioside lipid length and saturation state heterogeneity. These features of the lipid portion of the gangliosides can generate hundreds of permutations (e.g. Carbon chain lengths varying from 14-40, and unsaturation state ranging from 0 to 2). These features have never been elucidated in detail, and even less so for exploitation in diagnostics. Our early proof of concept data shows that we are able to identify unique signatures of lipid length and saturation which are specific and restricted to a specific cancer, and can be exploited as another diagnostic layer.

### Results

Using nLC-ESI-MS/MS (LC-MS), liquid biopsies from 6 cancer patients (2 Melanoma, 2 Renal cancer, 2 Small Cell Lung Cancer (SCLC newly diagnosed-treatment naïve) and 3 non-cancer controls (2 donors studied individually, and 1 pooled plasma from 30 donors) were analyzed. The LC-MS method was developed to study all tumor gangliosides at once (e.g. the Cancer Gangliosome Matrix, comprising 20 tumor gangliosides with at least 5,000 analytes).

Cancer patients had elevated TMGs and lipid tail heterogeneity patterns that appear to be specific to each cancer. Melanoma had significant increases in GM2 and GD3 and GD1. For GM2 the short chain forms increased 10-fold above normal, and long chain forms increased from undetectable in normal to 90 units in cancer. For GD3 the short chain forms increased 40-fold above normal. For GD1 short chain forms increased from undetectable in normal to 70 units. Renal cancer had significant increases in GD3 and GD2 (particularly in short lipid chain forms). Lung cancer had significant increases or shifts in GD3 (particularly in long lipid chain forms) and a shift in GM2 to short lipid chain forms. All control samples had signatures very low or below threshold, but normal gangliosides such as GM1 were detected. Internal standards (not shown) include Cholesterol and Phosphatidylcholine which are present at high levels in all samples. Phosphatidylserine (PS), is a non-specific positive marker of general stress (cancer, inflammation, diabetes, infection, sepsis, apoptosis).

Melanoma, renal cancer, lung cancer, and non-cancer is 1 patient per case, with relative quantification (Table 1). The limit of Quantifiable Detection (LOQD) was ~1-20 units.

In another LC-MS study, 12/13 OC serum samples had significant increases in GD3 compared to 2 non-cancer serum samples. Controls were very low or below threshold (Fig. 2).

These studies demonstrate the potential of LC-MS to quantify GD2/GD3. Our findings demonstrate an upregulation in the expression of GD3 during both stages and suggest its utility in the early screening of ovarian cancer.

In both data sets, the tumor gangliosides in serum differ quantitatively in terms of total quantification depending on whether non-cancer or cancer samples are evaluated (with TMGs being elevated in cancer).

### GD3(d18:1/23:0) -an important biomarker for Ovarian Cancer.

To address the need to identify the molecular identities of lipoforms, we used a reversed-phase nanobore liquid chromatography-electrospray ionization tandem mass spectrometry (nLC-ESI-MS/MS) method for GD2 and GD3. Interestingly, 70% of the GD3 in the serum of OC patients have only one type of relatively short lipid chains (Table 4).

This feature can be exploited to differentiate one cancer from another. Further bioinformatic analyses suggests that GD3 in the serum of 12 out of the 13 OC patients (Fig. 2) have discrete and highly restricted lipid chain lengths, whereas other gangliosides are highly heterogeneous chains length combinations. This underscores the importance for LC-MS analyses in asymptomatic screening. Remarkably, of the more than 500 possible lipoform permutations, the GD3(d18:1/23:0) isoform was the most represented, making up 70% of the total GD3 mass in 12 of the 13 OC patients, (127/181; p = 0.0005) (Table 4).

The results presented herein illustrate exemplary data regarding a pan-cancer liquid biopsy test for multiple indications for the detection of TMGs isolated from patient's serum. Through utilizing the signature of lipid tail heterogeneity, tumor of origin can be identified. This is a ground-breaking new type of cancer screening that utilizes advances in lipidomic science to detect specific cancer signals (variations in tumor marker gangliosides). Our new approach will set the stage for a new cancer screening paradigm with potentially higher specificity and clinical useful sensitivity compared to existing technologies.

These data further establish a pan-cancer early detection screening test. Our liquid biopsy technology provide cancer screening at a population level with a tremendous potential of helping people access more early effective treatment and substantially reducing overall cancer mortality.

### References

1. Vogelstein B, Papadopoulos N, Velculescu VE, Zhou S, Diaz LA, Kinzler KW. Cancer Genome Landscapes. Science. 2013;339(6127):1546-1558. doi:10.1126/science.1235122
2. Yachida S, White CM, Naito Y, et al. Clinical Significance of the Genetic Landscape of Pancreatic Cancer and Implications for Identification of Potential Long-term Survivors. Clin Cancer Res. 2012;18(22):6339-6347. doi:10.1158/1078-0432.CCR-12-1215
3. Cohen JD, Li L, Wang Y, et al. Detection and localization of surgically resectable cancers with a multi-analyte blood test. Science. 2018;359(6378):926-930. doi:10.1126/science.aar3247
4. Ahlquist DA. Universal cancer screening: revolutionary, rational, and realizable. Npj Precis Oncol. 2018;2(1):23. doi:10.1038/s41698-018-0066-x
5. Chen X, Gole J, Gore A, et al. Non-invasive early detection of cancer four years before conventional diagnosis using a blood test. Nat Commun. 2020;11(1):3475. doi:10.1038/s41467-020-17316-z
6. Lennon AM, Buchanan AH, Kinde I, et al. Feasibility of blood testing combined with PET-CT to screen for cancer and guide intervention. Science. 2020;369(6499):eabb9601. doi:10.1126/science.abb9601
7. Liu MC, Oxnard GR, Klein EA, et al. Sensitive and specific multi-cancer detection and localization using methylation signatures in cell-free DNA. Ann Oncol. 2020;31(6):745-759. doi:10.1016/j.annonc.2020.02.011
8. Constantin N, Sina AAI, Korbie D, Trau M. Opportunities for Early Cancer Detection: The Rise of ctDNA Methylation-Based Pan-Cancer Screening Technologies. Epigenomes. 2022;6(1):6. doi:10.3390/epigenomes6010006
9. Hubbell E, Clarke CA, Aravanis AM, Berg CD. Modeled Reductions in Late-stage Cancer with a Multi-Cancer Early Detection Test. Cancer Epidemiol Biomarkers Prev. 2021;30(3):460-468. doi:10.1158/1055-9965.EPI-20-1134
10. Varki A. Biological roles of glycans. Glycobiology. 2017;27(1):3-49. doi:10.1093/glycob/cww086
11. Bartish M, del Rincón SV, Rudd CE, Saragovi HU. Aiming for the Sweet Spot: Glyco-Immune Checkpoints and γδ T Cells in Targeted Immunotherapy. Front Immunol. 2020;11:564499. doi:10.3389/fimmu.2020.564499
12. Möckl L. The Emerging Role of the Mammalian Glycocalyx in Functional Membrane Organization and Immune System Regulation. Front Cell Dev Biol. 2020;8:253. doi:10.3389/fcell.2020.00253
13. Pike LJ. Lipid rafts: bringing order to chaos. J Lipid Res. 2003;44(4):655-667. doi:10.1194/jlr.R200021-JLR200
14. Glycolipids - Latest research and news | Nature. Accessed November 10, 2021. https://www.nature.com/subjects/glycolipids#research-and-reviews
15. Schnaar RL, Kinoshita T. Glycosphingolipids. In: Varki A, Cummings RD, Esko JD, et al., eds. Essentials of Glycobiology. 3rd ed. Cold Spring Harbor Laboratory Press; 2015. Accessed November 10, 2021. http://www.ncbi.nlm.nih.gov/books/NBK453016/
16. Saragovi HU, Gagnon M. Gangliosides: therapeutic agents or therapeutic targets? Expert Opin Ther Pat. 2002;12(8):1215-1223. doi:10.1517/13543776.12.8.1215
17. Daniotti JL, Lardone RD, Vilcaes AA. Dysregulated Expression of Glycolipids in Tumor Cells: From Negative Modulator of Anti-tumor Immunity to Promising Targets for Developing Therapeutic Agents. Front Oncol. 2016;5. doi:10.3389/fonc.2015.00300
18. Zhang X, Kiechle FL. Review: Glycosphingolipids in health and disease. Ann Clin Lab Sci. 2004;34(1):3-13.
19. Regina Todeschini A, Hakomori S itiroh. Functional role of glycosphingolipids and gangliosides in control of cell adhesion, motility, and growth, through glycosynaptic microdomains. Biochim Biophys Acta BBA - Gen Subj. 2008;1780(3):421-433. doi:10.1016/j.bbagen.2007.10.008
20. Navid F, Santana VM, Barfield RC. Anti-GD2 antibody therapy for GD2-expressing tumors. Curr Cancer Drug Targets. 2010;10(2):200-209. doi:10.2174/156800910791054167
21. Cheever MA, Allison JP, Ferris AS, et al. The Prioritization of Cancer Antigens: A National Cancer Institute Pilot Project for the Acceleration of Translational Research. Clin Cancer Res. 2009;15(17):5323-5337. doi:10.1158/1078-0432.CCR-09-0737
22. Yang RK, Sondel PM. Anti-GD2 strategy in the treatment of neuroblastoma. Drugs Future. 2010;35(8):665. doi:10.1358/dof.2010.035.08.1513490
23. Elkashef SM, Allison SJ, Sadiq M, et al. Polysialic acid sustains cancer cell survival and migratory capacity in a hypoxic environment. Sci Rep. 2016;6(1):33026. doi:10.1038/srep33026
24. Hettmer S, Ladisch S, Kaucic K. Low complex ganglioside expression characterizes human neuroblastoma cell lines. Cancer Lett. 2005;225(1):141-149. doi:10.1016/j.canlet.2004.11.036
25. Mujoo K, Cheresh DA, Yang HM, Reisfeld RA. Disialoganglioside GD2 on human neuroblastoma cells: target antigen for monoclonal antibody-mediated cytolysis and suppression of tumor growth. Cancer Res. 1987;47(4):1098-1104.
26. Cheresh DA, Pierschbacher MD, Herzig MA, Mujoo K. Disialogangliosides GD2 and GD3 are involved in the attachment of human melanoma and neuroblastoma cells to extracellular matrix proteins. J Cell Biol. 1986;102(3):688-696. doi:10.1083/jcb.102.3.688
27. D. Kozireski-Chuback, G. Wu, and R.W. Ledeen, Developmental appearance of nuclear GM1 in neurons of the central and peripheral nervous systems. Brain Res Dev Brain Res 115 (1999) 201-8.
28. Cavanna B, Carpo M, Pedotti R, et al. Anti-GM2 IgM antibodies: clinical correlates and reactivity with a human neuroblastoma cell line. J Neuroimmunol. 1999;94(1-2):157-164. doi:10.1016/S0165-5728(98)00245-8
29. Vrionis FD, Wikstrand CJ, Fredman P, Månsson JE, Svennerholm L, Bigner DD. Five new epitope-defined monoclonal antibodies reactive with GM2 and human glioma and medulloblastoma cell lines. Cancer Res. 1989;49(23):6645-6651.
30. Mirkin BL, Clark SH, Zhang C. Inhibition of human neuroblastoma cell proliferation and EGF receptor phosphorylation by gangliosides GM 1 , GM 3 , GD 1A and GT 1B: Inhibition of neuroblastoma cell proliferation by gangliosides. Cell Prolif. 2002;35(2):105-115. doi:10.1046/j.1365-2184.2002.00228.x
31. Hildebrandt H, Becker C, Glüer S, Rösner H, Gerardy-Schahn R, Rahmann H. Polysialic acid on the neural cell adhesion molecule correlates with expression of polysialyltransferases and promotes neuroblastoma cell growth. Cancer Res. 1998;58(4):779-784.
32. Valentiner U. Expression of the neural cell adhesion molecule and polysialic acid in human neuroblastoma cell lines. Int J Oncol. Published online May 10, 2011. doi:10.3892/ijo.2011.1038
33. Pukel CS, Lloyd KO, Travassos LR, Dippold WG, Oettgen HF, Old LJ. GD3, a prominent ganglioside of human melanoma. Detection and characterisation by mouse monoclonal antibody. J Exp Med. 1982;155(4):1133-1147. doi:10.1084/jem.155.4.1133
34. Nicolae CD, Nicolae I. Antibodies against GM1 gangliosides associated with metastatic melanoma. Acta Dermatovenerol Croat ADC. 2013;21(2):86-92.
35. Hoon DSB, Ando I, Sviland G, et al. Ganglioside GM2 expression on human melanoma cells correlates with sensitivity to lymphokine-activated killer cells. Int J Cancer. 1989;43(5):857-862. doi:10.1002/ijc.2910430520
36. Tsuchida T, Saxton RE, Morton DL, Irie RF. Gangliosides of human melanoma. Cancer. 1989;63(6):1166-1174. doi:10.1002/1097-0142(19890315)63:6<1166::aid-cncr2820630621>3.0.co;2-5
37. Mennel HD, Bosslet K, Wiegandt H, Sedlacek HH, Bauer BL, Rodden AF. Expression of GD2-epitopes in human intracranial tumors and normal brain. Exp Toxicol Pathol. 1992;44(6):317-324. doi:10.1016/S0940-2993(11)80218-6
38. Lama G, Mangiola A, Proietti G, et al. Progenitor/Stem Cell Markers in Brain Adjacent to Glioblastoma: GD3 Ganglioside and NG2 Proteoglycan Expression. J Neuropathol Exp Neurol. 2016;75(2):134-147. doi:10.1093/jnen/nlv012
39. Ohkawa Y, Momota H, Kato A, et al. Ganglioside GD3 Enhances Invasiveness of Gliomas by Forming a Complex with Platelet-derived Growth Factor Receptor α and Yes Kinase. J Biol Chem. 2015;290(26):16043-16058. doi:10.1074/jbc.M114.635755
40. Birks SM, Danquah JO, King L, Vlasak R, Gorecki DC, Pilkington GJ. Targeting the GD3 acetylation pathway selectively induces apoptosis in glioblastoma. Neuro-Oncol. 2011;13(9):950-960. doi:10.1093/neuonc/nor108
41. Hedberg KM, Mahesparan R, Read TA, et al. The glioma-associated gangliosides 3'-isoLM1, GD3 and GM2 show selective area expression in human glioblastoma xenografts in nude rat brains: Distribution of gangliosides in human glioblastoma xenografts. Neuropathol Appl Neurobiol. 2001;27(6):451-464. doi:10.1046/j.1365-2990.2001.00353.x
42. Fujimoto Y, Izumoto S, Suzuki T, et al. Ganglioside GM3 inhibits proliferation and invasion of glioma. J Neurooncol. 2005;71(2):99-106. doi:10.1007/s11060-004-9602-3
43. Noll ENE, Lin J, Nakatsuji Y, Miller RH, Black PMcL. GM3 as a Novel Growth Regulator for Human Gliomas. Exp Neurol. 2001;168(2):300-309. doi:10.1006/exnr.2000.7603
44. Bassi R, Viani P, Giussani P, Riboni L, Tettamanti G. GM3 ganglioside inhibits endothelin-1-mediated signal transduction in C6 glioma cells. FEBS Lett. 2001;507(1):101-104. doi:10.1016/s0014-5793(01)02966-0
45. Trouillas J, Daniel L, Guigard MP, et al. Polysialylated neural cell adhesion molecules expressed in human pituitary tumors and related to extrasellar invasion. J Neurosurg. 2003;98(5):1084-1093. doi:10.3171/jns.2003.98.5.1084
46. Brezicka FT, Olling S, Nilsson O, et al. Immunohistological detection of fucosyl-GM1 ganglioside in human lung cancer and normal tissues with monoclonal antibodies. Cancer Res. 1989;49(5):1300-1305.
47. Yamada T, Bando H, Takeuchi S, et al. Genetically engineered humanized anti-ganglioside GM2 antibody against multiple organ metastasis produced by GM2-expressing small-cell lung cancer cells. Cancer Sci. 2011;102(12):2157-2163. doi:10.1111/j.1349-7006.2011.02093.x
48. Blanco R, Domínguez E, Morales O, et al. Prognostic Significance of N-Glycolyl GM3 Ganglioside Expression in Non-Small Cell Lung Carcinoma Patients: New Evidences. Pathol Res Int. 2015;2015:1-12. doi:10.1155/2015/132326
49. Hayashi N, Chiba H, Kuronuma K, et al. Detection of N-glycolyated gangliosides in non-small-cell lung cancer using GMR8 monoclonal antibody. Cancer Sci. 2013;104(1):43-47. doi:10.1111/cas.12027
50. Tanaka F, Otake Y, Nakagawa T, et al. Prognostic significance of polysialic acid expression in resected non-small cell lung cancer. Cancer Res. 2001;61(4):1666-1670.
51. Tanaka F, Otake Y, Nakagawa T, et al. Expression of polysialic acid and STX, a human polysialyltransferase, is correlated with tumor progression in non-small cell lung cancer. Cancer Res. 2000;60(11):3072-3080.
52. Liang JX, Liang Y, Gao W. Clinicopathological and prognostic significance of sialyl Lewis X overexpression in patients with cancer: a meta-analysis. OncoTargets Ther. 2016;9:3113-3125. doi:10.2147/OTT.S102389
53. Natoni A, Macauley MS, O'Dwyer ME. Targeting Selectins and Their Ligands in Cancer. Front Oncol. 2016;6. doi:10.3389/fonc.2016.00093
54. Yoshida S, Fukumoto S, Kawaguchi H, Sato S, Ueda R, Furukawa K. Ganglioside G(D2) in small cell lung cancer cell lines: enhancement of cell proliferation and mediation of apoptosis. Cancer Res. 2001;61(10):4244-4252.
55. Watarai S, Kiura K, Shigeto R, Shibayama T, Kimura I, Yasuda T. Establishment of Monoclonal Antibodies Specific for Ganglioside GM1: Detection of Ganglioside GM1 in Small Cell Lung Carcinoma Cell Lines and Tissues. J Biochem (Tokyo). 1994;116(5):948-954. doi:10.1093/oxfordjournals.jbchem.a124651
56. Liang YJ, Ding Y, Levery SB, Lobaton M, Handa K, Hakomori S i. Differential expression profiles of glycosphingolipids in human breast cancer stem cells vs. cancer non-stem cells. Proc Natl Acad Sci. 2013;110(13):4968-4973. doi:10.1073/pnas.1302825110
57. Steenackers A, Vanbeselaere J, Cazet A, et al. Accumulation of Unusual Gangliosides GQ3 and GP3 in Breast Cancer Cells Expressing the GD3 Synthase. Molecules. 2012;17(8):9559-9572. doi:10.3390/molecules17089559
58. Bobowski M, Vincent A, Steenackers A, et al. Estradiol Represses the GD3 Synthase Gene ST8SIA1 Expression in Human Breast Cancer Cells by Preventing NFκB Binding to ST8SIA1 Promoter. Migliaccio A, ed. PLoS ONE. 2013;8(4):e62559. doi: 10.13711journal.pone.0062559
59. Wang X, Li X, Zeng YN, He F, Yang XM, Guan F. Enhanced expression of polysialic acid correlates with malignant phenotype in breast cancer cell lines and clinical tissue samples. Int J Mol Med. 2016;37(1):197-206. doi:10.3892/ijmm.2015.2395
60. Das T, Sa G, Hilston C, et al. GM1 and tumor necrosis factor-alpha, overexpressed in renal cell carcinoma, synergize to induce T-cell apoptosis. Cancer Res. 2008;68(6):2014-2023. doi:10.1158/0008-5472.CAN-07-6037
61. Biswas K, Richmond A, Rayman P, et al. GM2 Expression in Renal Cell Carcinoma: Potential Role in Tumor-Induced T-Cell Dysfunction. Cancer Res. 2006;66(13):6816-6825. doi:10.1158/0008-5472.CAN-06-0250
62. Sakakibara N, Gasa S, Kamio K, et al. Distinctive glycolipid patterns in Wilms' tumor and renal cell carcinoma. Cancer Lett. 1991;57(3):187-192. doi:10.1016/0304-3835(91)90155-B
63. Borzym-Kluczyk M, Radziejewska I, Cechowska-Pasko M. Increased expression of MUC1 and sialyl Lewis antigens in different areas of clear renal cell carcinoma. Clin Exp Nephrol. 2015;19(4):732-737. doi:10.1007/s10157-014-1013-y
64. Ravindranath MH, Muthugounder S, Presser N, et al. Immunogenic gangliosides in human ovarian carcinoma. Biochem Biophys Res Commun. 2007;353(2):251-258. doi:10.1016/j.bbrc.2006.12.001
65. Webb TJ, Li X, Giuntoli RL, et al. Molecular Identification of GD3 as a Suppressor of the Innate Immune Response in Ovarian Cancer. Cancer Res. 2012;72(15):3744-3752. doi:10.1158/0008-5472.CAN-11-2695
66. Prinetti A, Aureli M, Illuzzi G, et al. GM3 synthase overexpression results in reduced cell motility and in caveolin-1 upregulation in human ovarian carcinoma cells. Glycobiology. 2010;20(1):62-77. doi:10.1093/glycob/cwp143
67. Chang HR, Cordon-Cardo C, Houghton AN, Cheung NK, Brennan MF. Expression of disialogangliosides GD2 and GD3 on human soft tissue sarcomas. Cancer. 1992;70(3):633-638. doi:10.1002/1097-0142(19920801)70:3<633::aid-cncr2820700315>3.0.co;2-f
68. Heiner JP, Miraldi F, Kallick S, et al. Localization of GD2-specific monoclonal antibody 3F8 in human osteosarcoma. Cancer Res. 1987;47(20):5377-5381.
69. Shibuya H, Hamamura K, Hotta H, et al. Enhancement of malignant properties of human osteosarcoma cells with disialyl gangliosides GD2/GD3. Cancer Sci. 2012;103(9):1656-1664. doi:10.1111/j.1349-7006.2012.02344.x
70. Dobrenkov K, Ostrovnaya I, Gu J, Cheung IY, Cheung NKV. Oncotargets GD2 and GD3 are highly expressed in sarcomas of children, adolescents, and young adults. Pediatr Blood Cancer. 2016;63(10):1780-1785. doi:https://doi.org/10.1002/pbc.26097
71. Lipinski M, Braham K, Philip I, et al. Neuroectoderm-associated antigens on Ewing's sarcoma cell lines. Cancer Res. 1987;47(1):183-187.
72. Kailayangiri S, Altvater B, Meltzer J, et al. The ganglioside antigen GD2 is surface-expressed in Ewing sarcoma and allows for MHC-independent immune targeting. Br J Cancer. 2012;106(6):1123-1133. doi:10.1038/bjc.2012.57
73. Scursoni AM, Galluzzo L, Camarero S, et al. Detection of N-Glycolyl GM3 Ganglioside in Neuroectodermal Tumors by Immunohistochemistry: An Attractive Vaccine Target for Aggressive Pediatric Cancer. Clin Dev Immunol. 2011;2011:1-6. doi:10.1155/2011/245181
74. Modak S, Gerald W, Cheung NKV. Disialoganglioside GD2 and a novel tumor antigen: Potential targets for immunotherapy of desmoplastic small round cell tumor. Med Pediatr Oncol. 2002;39(6):547-551. doi:10.1002/mpo.10151
75. Portoukalian J, David MJ, Richard M, Gain P. Shedding of GD2 ganglioside in patients with retinoblastoma. Int J Cancer. 2009;53(6):948-951. doi:10.1002/ijc.2910530614
76. Torbidoni AV, Scursoni A, Camarero S, et al. Immunoreactivity of the 14F7 Mab raised against N -Glycolyl GM3 Ganglioside in retinoblastoma tumours. Acta Ophthalmol (Copenh). 2015;93(4):e294-e300. doi:10.1111/aos.12578
77. Scursoni AM, Galluzzo L, Camarero S, et al. Detection and Characterization of N-Glycolyated Gangliosides in Wilms Tumor by Immunohistochemistry. Pediatr Dev Pathol. 2010;13(1):18-23. doi:10.2350/08-10-0544.1
78. Roth J, Zuber C, Wagner P, Blaha I, Bitter-Suermann D, Heitz PU. Presence of the long chain form of polysialic acid of the neural cell adhesion molecule in Wilms' tumor. Identification of a cell adhesion molecule as an oncodevelopmental antigen and implications for tumor histogenesis. Am J Pathol. 1988;133(2):227-240.
79. Komminoth P, Roth J, Saremaslani P, Matias-Guiu X, Wolfe HJ, Heitz PU. Polysialic acid of the neural cell adhesion molecule in the human thyroid: a marker for medullary thyroid carcinoma and primary C-cell hyperplasia. An immunohistochemical study on 79 thyroid lesions. Am J Surg Pathol. 1994;18(4):399-411. doi:10.1097/00000478-199404000-00008
80. Zhang S, Cordon-Cardo C, Zhang HS, et al. Selection of tumor antigens as targets for immune attack using immunohistochemistry: I. Focus on gangliosides. Int J Cancer. 1997;73(1):42-49. doi:10.1002/(sici)1097-0215(19970926)73:1<42::aid-ijc8>3.0.co;2-1
81. Santin AD, Ravindranath MH, Bellone S, et al. Increased levels of gangliosides in the plasma and ascitic fluid of patients with advanced ovarian cancer. BJOG Int J Obstet Gynaecol. 2004;111(6):613-618. doi:10.1111/j.1471-0528.2004.00142.x
82. Kong Y, Li R, Ladisch S. Natural forms of shed tumor gangliosides. Biochim Biophys Acta BBA - Lipids Lipid Metab. 1998;1394(1):43-56. doi:10.1016/S0005-2760(98)00096-4
83. Ladisch S, Li R, Olson E. Ceramide structure predicts tumor ganglioside immunosuppressive activity. Proc Natl Acad Sci. 1994;91(5):1974-1978. doi:10.1073/pnas.91.5.1974
84. Irani DN, Lin KI, Griffin DE. Brain-derived gangliosides regulate the cytokine production and proliferation of activated T cells. J Immunol Baltim Md 1950. 1996;157(10):4333-4340.
85. Wu AM, Khoo KH, Yu SY, Yang Z, Kannagi R, Watkins WM. Glycomic mapping of pseudomucinous human ovarian cyst glycoproteins: Identification of Lewis and sialyl Lewis glycotopes. PROTEOMICS. 2007;7(20):3699-3717. doi:10.1002/pmic.200700356
86. Ladisch S, Wu ZL. Detection of a Tumor-Associated Ganglioside in Plasma of Patients with Neuroblastoma. The Lancet. 1985;325(8421):136-138. doi:10.1016/S0140-6736(85)91906-3
87. Bernhard H, Zum Büschenfelde KHM, Dippold WG. Ganglioside GD3 shedding by human malignant melanoma cells. Int J Cancer. 1989;44(1):155-160. doi:10.1002/ijc.2910440127
88. Merritt WD, Der-Minassian V, Reaman GH. Increased GD3 ganglioside in plasma of children with T-cell acute lymphoblastic leukemia. Leukemia. 1994;8(5):816-822.
89. Valentino L, Moss T, Olson E, Wang H, Elashoff R, Ladisch S. Shed tumor gangliosides and progression of human neuroblastoma. Blood. 1990;75(7):1564-1567. doi:10.1182/blood.V75.7.1564.1564

### Incorporation by reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

Also incorporated by reference in their entirety are any polynucleotide and amino acid sequences which reference an accession number correlating to an entry in a public database, such as those maintained by The Institute for Genomic Research (TIGR) on the World Wide Web at tigr.org and/or the National Center for Biotechnology Information (NCBI) on the World Wide Web at ncbi.nlm.nih.gov.

### Equivalents

Those skilled in the art will recognize, or be able to ascertain using no more than routine experimentation, many equivalents to the specific embodiments of the present invention described herein. Such equivalents are intended to be encompassed by the following claims.

Additional preferred embodiments are as follows:
A preferred embodiment relateds to a method of detecting the presence, level, and/or lipid length of at least one ganglioside and/or at least one lipoform of a ganglioside, the method comprising detecting said ganglioside and/or at least one lipoform of a ganglioside in a sample using mass spectrometry, optionally wherein the sample is from a subject having cancer, a subject suspected of having cancer, or a cancer-free subject.

Another preferred embodiment is a method as above, wherein the mass spectrometry is selected from LC-ESI-MS/MS, LC-ESI-CID-MS/MS, nanobore LC-ESI-MS, and nanobore LC-ESI-MS/MS.

Another preferred embodiment is a a method of diagnosing a cancer in a subject, the method comprising:
a) determining the level of at least one ganglioside and/or at least one lipoform of a ganglioside in a subject sample using mass spectrometry as described herein; and
b) comparing said level of the at least one ganglioside and/or at least one lipoform of a ganglioside to that in a control sample,
wherein a significantly higher level of the at least one ganglioside and/or at least one lipoform of a ganglioside in the subject sample as compared to the control sample indicates that the subject has a cancer.

Another preferred embodiment is a method of determining a grade of a cancer, the method comprising:
a) determining the level of at least one ganglioside and/or at least one lipoform of a ganglioside in a subject sample using mass spectrometry as described herein; and
b) comparing said level of the at least one ganglioside and/or at least one lipoform of a ganglioside to that in a control sample,

wherein at least 100% and no more than 200% increase in the level of the at least one ganglioside and/or at least one lipoform of a ganglioside in the subject sample as compared to the level in the control sample indicates that the subject has a low grade cancer; and/or
wherein at least 200% increase in the level of the at least one ganglioside and/or at least one lipoform of a ganglioside in the subject sample as compared to the level in the control sample indicates that the subject has a high grade cancer.

Another preferred embodiment is a method of determining a tumor burden of a cancer, the method comprising:
a) determining the level of at least one ganglioside and/or at least one lipoform of a ganglioside in a subject sample using mass spectrometry as described herein; and
b) comparing said level of the at least one ganglioside and/or at least one lipoform of a ganglioside to that in a control sample,

wherein at least 100% and no more than 200% increase in the level of the at least one ganglioside and/or at least one lipoform of a ganglioside in the subject sample as compared to the level in the control sample indicates that the subject has a low tumor burden; and/or
wherein at least 200% increase in the level of the at least one ganglioside and/or at least one lipoform of a ganglioside in the subject sample as compared to the level in the control sample indicates that the subject has a high tumor burden.

Another preferred embodiment is a method of detecting a recurrence of a cancer in a subject, the method comprising:
a) obtaining or providing a sample from the subject whose cancer has regressed after receiving cancer treatment;
b) determining the level of at least one ganglioside and/or at least one lipoform of a ganglioside in the subject sample using mass spectrometry as described herein; and
c) comparing said level of the at least one ganglioside and/or at least one lipoform of a ganglioside to that in a control sample,
wherein a significantly higher level of the at least one ganglioside and/or at least one lipoform of a ganglioside in the subject sample as compared to the level in the control sample indicates a recurrence of a cancer in the subject.

Another preferred embodiment is a method of detecting a minimal residual disease in a subject, the method comprising:
a) obtaining or providing a sample from the subject in remission;
b) determining the level of at least one ganglioside and/or at least one lipoform of a ganglioside in the subject sample using mass spectrometry as described herein; and
c) comparing said level of the at least one ganglioside and/or at least one lipoform of a ganglioside to that in a control sample,
wherein a significantly higher level of the at least one ganglioside and/or at least one lipoform of a ganglioside in the subject sample as compared to the level in the control sample indicates that the subject has a minimal residual disease.
as described herein method of stratifying a subject afflicted with a cancer according to benefit from a cancer therapy, the method comprising:
a) determining the level of at least one ganglioside and/or at least one lipoform of a ganglioside in a sample from a subject administered with a cancer therapy using mass spectrometry as described herein;
b) determining the level of the at least one ganglioside and/or at least one lipoform of a ganglioside in a control; and
c) comparing the level of the at least one ganglioside and/or at least one lipoform of a ganglioside detected in steps a) and b);
wherein no significant change or a decrease in the level of the at least one ganglioside and/or at least one lipoform of a ganglioside in the subject sample as compared to the level in the control is an indication that the subject afflicted with the cancer would benefit from the cancer therapy.

Another preferred embodiment is a method of determining whether a subject afflicted with a cancer would likely respond to a cancer therapy, the method comprising:
a) determining the level of at least one ganglioside and/or at least one lipoform of a ganglioside in a sample from a subject administered with a cancer therapy using mass spectrometry as described herein;
b) determining the level of the at least one ganglioside and/or at least one lipoform of a ganglioside in a control; and
c) comparing the level of the at least one ganglioside and/or at least one lipoform of a ganglioside detected in steps a) and b);

wherein a significantly higher level of the at least one ganglioside and/or at least one lipoform of a ganglioside in the subject sample as compared to the level in the control is an indication that the subject afflicted with the cancer would not respond to the cancer therapy; and/or
wherein no significant change or a decrease in the level of the at least one ganglioside and/or at least one lipoform of a ganglioside in the subject sample as compared to the level in the control is an indication that the subject afflicted with the cancer would respond to the cancer therapy.

Another preferred embodiment is a method for predicting the clinical outcome of a subject afflicted with a cancer, the method comprising:
a) determining the level of at least one ganglioside and/or at least one lipoform of a ganglioside in a subject sample using mass spectrometry as described herein;
b) determining the level of the at least one ganglioside and/or at least one lipoform of a ganglioside in a control; and
c) comparing the level of the at least one ganglioside and/or at least one lipoform of a ganglioside determined in steps a) and b);
wherein a significantly higher level of the at least one ganglioside and/or at least one lipoform of a ganglioside in the subject sample as compared to the level in the control is an indication that the subject has a poor clinical outcome.

Another preferred embodiment is a method of monitoring the progression of a cancer in a subject, the method comprising:
a) detecting in a subject sample at a first point in time the level of at least one ganglioside and/or at least one lipoform of a ganglioside using mass spectrometry as described herein;
b) repeating step a) at a subsequent point in time; and
c) comparing the level of the at least one ganglioside and/or at least one lipoform of
a ganglioside detected in steps a) and b) to monitor the progression of the cancer in the subject, optionally wherein the subject is at risk for developing a cancer.

Another preferred embodiment a method as described herein, wherein between the first point in time and the subsequent point in time, the subject has received a cancer therapy.

Another preferred embodiment is a method of assessing the efficacy of a cancer therapy in a subject afflicted with a cancer, the method comprising:
a) determining the level of at least one ganglioside and/or at least one lipoform of a ganglioside using mass spectrometry as described herein, in a first sample obtained from a subject;
b) repeating step a) during at least one subsequent point in time after administration of the cancer therapy; and
c) comparing the level of at least one ganglioside and/or at least one lipoform of a ganglioside detected in steps a) and b),
wherein a significantly lower level of the at least one ganglioside and/or at least one lipoform of a ganglioside in the at least one subsequent sample, relative to the first sample, is an indication that the therapy is efficacious to treat a cancer in the subject.

Another preferred embodiment is a method as described herein, wherein the first and/or at least one subsequent sample is a portion of a single sample or pooled samples obtained from the subject.

Another preferred embodiment is a method of diagnosing a cancer in a subject, the method comprising:
a) determining the lipid length of at least one ganglioside in a subject sample using mass spectrometry as described herein; and
b) comparing the said lipid length of the at least one ganglioside to that in a control sample,
wherein a significant change in heterogeneity of the lipid length of the at least one ganglioside in the subject sample as compared to the control sample indicates that the subject has a cancer.

Another preferred embodiment is a method of determining a grade of a cancer, the method comprising:
a) determining the lipid length of at least one ganglioside in the subject sample using mass spectrometry as described herein; and
b) comparing the said lipid length of the at least one ganglioside to that in a control sample,

wherein at least 100% and no more than 200% change (e.g., increase or decrease) in heterogeneity of the lipid length of the at least one ganglioside in the subject sample as compared to that in the control sample indicates that the subject has a low grade cancer; and/or
wherein at least 200% change (e.g., increase or decrease) in heterogeneity of the lipid length of the at least one ganglioside in the subject sample as compared to that in the control sample indicates that the subject has a high grade cancer.

Another preferred embodiment is a method of determining a tumor burden of a cancer, the method comprising:
a) determining the lipid length of at least one ganglioside in the subject sample using mass spectrometry as described herein; and
b) comparing the said lipid length of the at least one ganglioside to that in a control sample,

wherein at least 100% and no more than 200% change (e.g., increase or decrease) in heterogeneity of the lipid length of the at least one ganglioside in the subject sample as compared to that in the control sample indicates that the subject has a low tumor burden; and/or
wherein at least 200% change (e.g., increase or decrease) in heterogeneity of the lipid length of the at least one ganglioside in the subject sample as compared to that in the control sample indicates that the subject has a high tumor burden.

Another preferred embodiment is a method of detecting a recurrence of a cancer in a subject, the method comprising:
a) obtaining or providing a sample from the subject whose cancer has regressed after receiving cancer treatment;
b) determining the lipid length of at least one ganglioside in the subject sample using mass spectrometry as described herein; and
c) comparing the said lipid length of the at least one ganglioside to that in a control sample,
wherein a significant change in heterogeneity of the lipid length of the at least one ganglioside in the subject sample as compared to that in the control sample indicates a recurrence of a cancer in a subject.

Another preferred embodiment is a method of detecting a minimal residual disease in a subject, the method comprising:
a) obtaining or providing a sample from the subject in remission;
b) determining the lipid length of at least one ganglioside in the subject sample using mass spectrometry as described herein; and
c) comparing the said lipid length of the at least one ganglioside to that in a control sample,
wherein a significant change in heterogeneity of the lipid length of the at least one ganglioside in the subject sample as compared to that in the control sample indicates that the subject has a minimal residual disease.

Another preferred embodiment is a method of stratifying a subject afflicted with a cancer according to benefit from a cancer therapy (e.g., immunotherapy), the method comprising:
a) determining the lipid length of at least one ganglioside in a sample from a subject administered with a cancer therapy using mass spectrometry as described herein;
b) determining the lipid length of the at least one ganglioside in a control; and
c) comparing the lipid length of the at least one ganglioside detected in steps a) and b);
wherein no significant change in heterogeneity of the lipid length of the at least one ganglioside in the subject sample as compared to that in the control is an indication that the subject afflicted with the cancer would benefit from the cancer therapy.

Another preferred embodiment is a method of determining whether a subject afflicted with a cancer would likely respond to a cancer therapy (e.g., immunotherapy), the method comprising:
a) determining the lipid length of at least one ganglioside in a sample from a subject administered with a cancer therapy using mass spectrometry as described herein;
b) determining the lipid length of the at least one ganglioside in a control; and
c) comparing the lipid length of the at least one ganglioside detected in steps a) and b);

wherein a significant change in heterogeneity of the lipid length of the at least one ganglioside in the subject sample as compared to that in the control is an indication that the subject afflicted with the cancer would not respond to the cancer therapy; and/or
wherein no significant change in heterogeneity of the lipid length of the at least one ganglioside in the subject sample as compared to that in the control is an indication that the subject afflicted with the cancer would respond to the cancer therapy.

Another preferred embodiment is a method for predicting the clinical outcome of a subject afflicted with a cancer, the method comprising:
a) determining the lipid length of at least one ganglioside in a subject sample using mass spectrometry as described herein;
b) determining the lipid length of the at least one ganglioside in a control; and
c) comparing the lipid length of the at least one ganglioside determined in steps a) and b);
wherein a significant change in heterogeneity of the lipid length of the at least one ganglioside in the subject sample as compared to the control sample is an indication that the subject has a poor clinical outcome.

Another preferred embodiment is a method of monitoring the progression of a cancer in a subject, the method comprising:
a) detecting in a subject sample at a first point in time the lipid length of at least one ganglioside using mass spectrometry as described herein;
b) repeating step a) at a subsequent point in time; and
c) comparing heterogeneity of the lipid length of the at least one ganglioside detected in steps a) and b) to monitor the progression of the cancer in the subject, optionally wherein the subject is at risk for developing a cancer.

Another preferred embodiment is a method as described herein, wherein between the first point in time and the subsequent point in time, the subject has received a cancer therapy.

Another preferred embodiment is a method of assessing the efficacy of a cancer therapy in a subject afflicted with a cancer, the method comprising:
a) determining the lipid length of at least one ganglioside using mass spectrometry as described herein, in a first sample obtained from the subject;
b) repeating step a) during at least one subsequent point in time after administration of the cancer therapy; and
wherein a significant change in heterogeneity of the lipid length of the at least one ganglioside in the second sample, relative to the first sample, is an indication that the therapy is efficacious to treat a cancer in the subject.

Another preferred embodiment is a method as described herein, wherein the first and/or at least one subsequent sample is a portion of a single sample or pooled samples obtained from the subject.

Another preferred embodiment is a method as described herein, further comprising treating the subject with a cancer therapy (e.g., recommending, prescribing, and/or administering to the subject a cancer therapy).

Another preferred embodiment is a method as described herein, wherein the cancer therapy is a surgery, chemotherapy, cancer vaccines, chimeric antigen receptors, radiation therapy, immunotherapy, a modulator of expression of immune checkpoint inhibitory proteins or ligands, or any combination thereof.

Another preferred embodiment is a method as described herein, wherein the immunotherapy is an immune checkpoint inhibition therapy.

Another preferred embodiment is a method as described herein, wherein the cancer therapy is avelumab, durvalumab, atezolizumab, BRAF/MEK inhibitor, pembrolizumab, nivolumab, ipilimumab, or a combination thereof.

Another preferred embodiment is a method as described herein, wherein the at least one ganglioside and/or at least one lipoform of a ganglioside is a tumor-associated ganglioside and/or a lipoform of a tumor-associated ganglioside.

Another preferred embodiment is a method as described herein, wherein the ganglioside comprises GD2, GD3, GD1b, GT1b, fucosyl-GM1, GloboH, polysialic acid (PSA), GM2, GM3, sialyl-Lewis^{X}, sialyl-Lewis^{Y}, sialyl-Lewis^{A}, sialyl-Lewis^{B}, Lewis^{Y}, any portion thereof, any lipoform thereof, or any combination thereof.

Another preferred embodiment is a method as described herein, wherein the at least one ganglioside and/or at least one lipoform of a ganglioside comprises GM2, GD3, GD2, GD1b, any lipoform thereof, or any combination thereof.

Another preferred embodiment is a method as described herein, wherein the at least one ganglioside and/or at least one lipoform of a ganglioside comprises a combination of two or more selected from GM2, GD3, GD2, GD1b, and any lipoform thereof.

Another preferred embodiment is a method as described herein, wherein the at least one ganglioside and/or the at least one lipoform of a ganglioside comprises GD3(d18:1/16:0), GD3(d18:1/23:0), GD3(d18:1/24:1), GD2(d18:1/16:0), GD1b(d18:1/16:0), GD1b(d18:1/24:1), GD1b(d18:1/18:1), or any combination of two or more thereof.

Another preferred embodiment is a method as described herein wherein the at least one ganglioside and/or the at least one lipoform of a ganglioside comprises GD3(d18:1/23:0) and/or GD3(d18:1/24:1).

Another preferred embodiment is a method as described herein, wherein the at least one ganglioside and/or the at least one lipoform of a ganglioside comprises GD3(d18:1/23:0).

Another preferred embodiment is a method as described herein, wherein the at least one ganglioside and/or the at least one lipoform of a ganglioside comprises an acyl chain 24:1.

Another preferred embodiment is a method as described herein, wherein the acyl chain 24:1 is present in GD3 and/or GD1b.

Another preferred embodiment is a method as described herein, wherein the cancer or tumor is selected from the group consisting of neuroblastoma, lymphoma, leukemia, melanoma, glioma, small cell lung cancer, breast carcinoma, ovarian cancer, soft tissue sarcomas, osteosarcoma, Ewing's sarcoma, desmoplastic round cell tumor, rhabdomyosarcoma, retinoblastoma, non-small cell lung cancer, renal cell cancer, Wilms tumor, prostate cancer, gastric cancer, endometrial cancer, pancreatic cancer, and colon cancer.

Another preferred embodiment is a method as described herein, wherein the cancer or tumor is ovarian cancer, melanoma, renal cancer, or lung cancer, optionally wherein the cancer or tumor is ovarian cancer.

Another preferred embodiment is a method as described herein, wherein the cancer is a borderline tumor.

Another preferred embodiment is a method as described herein, wherein the sample comprises cells, serum, blood, peritumoral tissue, and/or intratumoral tissue obtained from the subject.

Another preferred embodiment is a method as described herein, wherein the sample comprises serum or blood.

Another preferred embodiment is a method as described herein, wherein said significantly higher level of at least one ganglioside comprises an at least twenty percent increase of the level of the at least one ganglioside.

Another preferred embodiment is method as described herein, wherein the significantly lower level of at least one ganglioside comprises an at least twenty percent decrease of the level of the at least one ganglioside.

Another preferred embodiment is method as described herein, wherein the control sample is a sample from a cancer-free subject.

Another preferred embodiment is method as described herein, wherein the control sample is a sample from the subject (e.g., first sample collected from the subject in longitudinal collections, to evaluate changes over time of the level of the at least one ganglioside).

Another preferred embodiment is method as described herein, wherein the subject is a mammal (e.g., afflicted with a cancer, e.g., a subject who is asymptomatic).

Another preferred embodiment is method as described herein, wherein the subject is an animal model of cancer, a dog, a cat, or a human.

Another preferred embodiment is method as described herein, wherein the subject is a human.

## Claims

1. A method of detecting the presence, level, and/or lipid length of at least one ganglioside and/or at least one lipoform of a ganglioside, the method comprising detecting said ganglioside and/or at least one lipoform of a ganglioside in a sample using mass spectrometry,
wherein the at least one ganglioside and/or the at least one lipoform of a ganglioside comprises GD3(d18:1/16:0), GD3(d18:1/23:0), GD3(d18:1/24:1), GD2(d18:1/16:0), GD1b(d18:1/16:0), GD1b(d18:1/24:1), GD1b(d18:1/18:1), or any combination of two or more thereof,
preferably wherein the mass spectrometry is selected from LC-ESI-MS/MS, LC-ESI-CID-MS/MS, nanobore LC-ESI-MS, and nanobore LC-ESI-MS/MS.

2. A method of diagnosing a cancer in a subject, the method comprising:
a) determining the level of at least one ganglioside and/or at least one lipoform of a ganglioside in a subject sample using mass spectrometry according to claim 1; and
b) comparing said level of the at least one ganglioside and/or at least one lipoform of a ganglioside to that in a control sample,
wherein a significantly higher level of the at least one ganglioside and/or at least one lipoform of a ganglioside in the subject sample as compared to the control sample indicates that the subject has a cancer.

3. A method of determining a grade of a cancer or a tumor burden of a cancer, the method comprising:
a) determining the level of at least one ganglioside and/or at least one lipoform of a ganglioside in a subject sample using mass spectrometry according to claim 1; and
b) comparing said level of the at least one ganglioside and/or at least one lipoform of a ganglioside to that in a control sample,
wherein at least 100% and no more than 200% increase in the level of the at least one ganglioside and/or at least one lipoform of a ganglioside in the subject sample as compared to the level in the control sample indicates that the subject has a low grade cancer or a low tumor burden; and/or
wherein at least 200% increase in the level of the at least one ganglioside and/or at least one lipoform of a ganglioside in the subject sample as compared to the level in the control sample indicates that the subject has a high grade cancer or a high tumor burden.

4. A method of detecting a recurrence of a cancer in a subject, the method comprising:
a) obtaining or providing a sample from the subject whose cancer has regressed after receiving cancer treatment;
b) determining the level of at least one ganglioside and/or at least one lipoform of a ganglioside in the subject sample using mass spectrometry according to claim 1; and
c) comparing said level of the at least one ganglioside and/or at least one lipoform of a ganglioside to that in a control sample,
wherein a significantly higher level of the at least one ganglioside and/or at least one lipoform of a ganglioside in the subject sample as compared to the level in the control sample indicates a recurrence of a cancer in the subject.

5. A method of detecting a minimal residual disease in a subject, the method comprising:
a) obtaining or providing a sample from the subject in remission;
b) determining the level of at least one ganglioside and/or at least one lipoform of a ganglioside in the subject sample using mass spectrometry according to claim 1; and
c) comparing said level of the at least one ganglioside and/or at least one lipoform of a ganglioside to that in a control sample,
wherein a significantly higher level of the at least one ganglioside and/or at least one lipoform of a ganglioside in the subject sample as compared to the level in the control sample indicates that the subject has a minimal residual disease.

6. A method of stratifying a subject afflicted with a cancer according to benefit from a cancer therapy, the method comprising:
a) determining the level of at least one ganglioside and/or at least one lipoform of a ganglioside in a sample from a subject administered with a cancer therapy using mass spectrometry according to claim 1;
b) determining the level of the at least one ganglioside and/or at least one lipoform of a ganglioside in a control; and
c) comparing the level of the at least one ganglioside and/or at least one lipoform of a ganglioside detected in steps a) and b);
wherein no significant change or a decrease in the level of the at least one ganglioside and/or at least one lipoform of a ganglioside in the subject sample as compared to the level in the control is an indication that the subject afflicted with the cancer would benefit from the cancer therapy.

7. A method of determining whether a subject afflicted with a cancer would likely respond to a cancer therapy, the method comprising:
a) determining the level of at least one ganglioside and/or at least one lipoform of a ganglioside in a sample from a subject administered with a cancer therapy using mass spectrometry according to claim 1;
b) determining the level of the at least one ganglioside and/or at least one lipoform of a ganglioside in a control; and
c) comparing the level of the at least one ganglioside and/or at least one lipoform of a ganglioside detected in steps a) and b);
wherein a significantly higher level of the at least one ganglioside and/or at least one lipoform of a ganglioside in the subject sample as compared to the level in the control is an indication that the subject afflicted with the cancer would not respond to the cancer therapy; and/or
wherein no significant change or a decrease in the level of the at least one ganglioside and/or at least one lipoform of a ganglioside in the subject sample as compared to the level in the control is an indication that the subject afflicted with the cancer would respond to the cancer therapy.

8. A method for predicting the clinical outcome of a subject afflicted with a cancer, the method comprising:
a) determining the level of at least one ganglioside and/or at least one lipoform of a ganglioside in a subject sample using mass spectrometry according to claim 1;
b) determining the level of the at least one ganglioside and/or at least one lipoform of a ganglioside in a control; and
c) comparing the level of the at least one ganglioside and/or at least one lipoform of a ganglioside determined in steps a) and b);
wherein a significantly higher level of the at least one ganglioside and/or at least one lipoform of a ganglioside in the subject sample as compared to the level in the control is an indication that the subject has a poor clinical outcome.

9. A method of monitoring the progression of a cancer in a subject, the method comprising:
a) detecting in a subject sample at a first point in time the level of at least one ganglioside and/or at least one lipoform of a ganglioside using mass spectrometry according to claim 1;
b) repeating step a) at a subsequent point in time; and
c) comparing the level of the at least one ganglioside and/or at least one lipoform of a ganglioside detected in steps a) and b) to monitor the progression of the cancer in the subject,
preferably wherein (a) the subject is at risk for developing a cancer, and/or (b) between the first point in time and the subsequent point in time, the subject has received a cancer therapy.

10. A method of assessing the efficacy of a cancer therapy in a subject afflicted with a cancer, the method comprising:
a) determining the level of at least one ganglioside and/or at least one lipoform of a ganglioside using mass spectrometry according to claim 1, in a first sample obtained from a subject;
b) repeating step a) during at least one subsequent point in time after administration of the cancer therapy; and
c) comparing the level of at least one ganglioside and/or at least one lipoform of a ganglioside detected in steps a) and b),
wherein a significantly lower level of the at least one ganglioside and/or at least one lipoform of a ganglioside in the at least one subsequent sample, relative to the first sample, is an indication that the therapy is efficacious to treat a cancer in the subject.

11. The method of claim 9 or 10, wherein the first and/or at least one subsequent sample is a portion of a single sample or pooled samples obtained from the subject.

12. The method of any one of claims 1-11, wherein
(a) the sample is from a subject having cancer, a subject suspected of having cancer, or a cancer-free subject; and/or
(b) the sample comprises cells, serum, blood, peritumoral tissue, and/or intratumoral tissue obtained from the subject.

13. The method of any one of claims 2-12, wherein said significantly higher level or significantly lower level of at least one ganglioside comprises an at least twenty percent increase or at least twenty percent decrease of the level of the at least one ganglioside, repsectively.

14. The method of any one of claims 2-13, wherein
(a) the control sample is a sample from a cancer-free subject; or
(b) the control sample is a sample from the subject (e.g., first sample collected from the subject in longitudinal collections, to evaluate changes over time of the level of the at least one ganglioside).

15. The method of any one of claims 2-14, wherein the subject is a mammal, a subject afflicted with a cancer, a subject who is asymptomatic, an animal model of cancer, a dog, a cat, or a human.
